(19) Europäisches Patentamt
European Patent Office
Office européen des brevets

(11) Numéro de publication: **0 259 197 B1**

(12) # FASCICULE DE BREVET EUROPEEN

(45) Date de publication de fascicule du brevet: **25.09.91**

(51) Int. Cl.[5]: **A61K 37/14**, C07K 17/02

(21) Numéro de dépôt: **87401559.7**

(22) Date de dépôt: **02.07.87**

Le dossier contient des informations techniques présentées postérieurement au dépôt de la demande et ne figurant pas dans le présent fascicule.

(54) **Conjugués macromuléculaires d'hémoglobine leur procédé de préparation et leurs applications.**

(30) Priorité: **02.07.86 FR 8609625**

(43) Date de publication de la demande: **09.03.88 Bulletin 88/10**

(45) Mention de la délivrance du brevet: **25.09.91 Bulletin 91/39**

(84) Etats contractants désignés: **AT BE CH DE ES FR GB GR IT LI LU NL SE**

(56) Documents cités:
**EP-A- 0 043 675**
**EP-A- 0 067 029**
**EP-A- 0 142 125**
**FR-A- 2 328 478**
**FR-A- 2 551 660**

**CHEMICAL ABSTRACTS, vol. 100, no. 8, 20 février 1984, page 318, résumé no. 56721m, Columbus, Ohio, US; E. DELLACHERIE et al.: "Modification of human hemoglobin by covalent association with soluble dextran", & BIOCHIM. BIOPHYS. ACTA 1983, 749(1), 106-14**

(73) Titulaire: **PASTEUR MERIEUX Sérums et Vaccins**
**58, avenue Leclerc**
**F-69007 Lyon(FR)**

(72) Inventeur: **Leonard, Michèle**
**2 rue Pierre Curie**
**F-54500 Vandoeuvre(FR)**
Inventeur: **Dellacherie, Edith**
**15 rue Jean Ploussard**
**F-54220 Malzeville(FR)**
Inventeur: **Neel, Jean Maurice Léon**
**24 avenue du Château**
**F-54600 Villers Les Nancy(FR)**
Inventeur: **Vigneron, Claude**
**60 quai Claude Le Lorrain**
**F-54000 Nancy(FR)**
Inventeur: **Labrude, Pierre**
**72 rue du Petit Arbois**
**F-54520 Laxou(FR)**
Inventeur: **Bonneaux, François**
**29 rue Emile Gallé**
**F-54000 Nancy(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 259 197 B1

Rank Xerox (UK) Business Services

CHEMICAL ABSTRACTS, vol. 104, no. 23, 9 juin 1986, page 331, résumé no. 202641m, Columbus, Ohio, US; D. SACCO et al.: "Interaction of a macromolecular polyanion, dextran sulfate, with human hemoglobin", & FEBS LETT. 1986, 199(2), 254-8

Inventeur: Sacco, Daniel
11 boulevard Charles V
F-54000 Nancy(FR)

(74) Mandataire: Grosset-Fournier, Chantal Catherine et al
SC Ernest Gutmann/Yves Plasseraud 67 boulevard Haussmann
F-75008 Paris(FR)

## Description

L'invention concerne de nouveaux conjugués macromoléculaires, leur procédé de préparation et leurs applications comme transporteurs d'oxygène, notamment dans le cadre des transfusions.

On sait qu'il est possible d'injecter par voie intraveineuse une solution aqueuse d'hémoglobine exempte de stroma et rendue isotonique du sang.

Or l'un des inconvénients que présente l'hémoglobine est qu'elle ne reste pas dans le circuit sanguin mais qu'elle diffuse à l'extérieur du système vasculaire, en raison en particulier de sa petite taille.

Afin de remédier à cet inconvénient, c'est à dire afin d'augmenter la persistance intravasculaire de l'hémoglobine libre dans le cadre de son utilisation comme "substitut" du sang ou de soluté de remplissage transporteur d'oxygène, plusieurs procédés ont été utilisés.

On a, par exemple, fixé l'hémoglobine à des macromolécules hydrosolubles dépourvues de toxicité, non antigéniques et hémocompatibles.

C'est ainsi que de nombreux exemples d'hémoglobines modifiées par la fixation chimique de polymères hydrosolubles montrent que le temps de séjour de l'hémoglobine dans l'organisme peut être largement augmenté.

Parmi les différents polymères utilisés, les plus courants sont les polysaccharides et notamment le dextrane (brevet français n° 2.328.478) l'hydroxyéthylamidon (brevet français n° 2.328.478), l'inuline (demande de brevet européen n° 43.675), et les polyalkylèneglycols et plus particulièrement le polyéthylène glycol.

Cependant dans tous les cas, l'hémoglobine ainsi modifiée par fixation directe de ces polymères, possède des propriétés oxyphoriques mal adaptées à une utilisation en transfusion sanguine.

On rappelle, en effet, qu'un substitut sanguin ne peut jouer un rôle équivalent à celui qu'assume l'hémoglobine intraérythrocytaire native que dans la mesure où la combinaison qu'il forme avec l'oxygène est réversible, c'est à dire qu'il est capable de fixer l'oxygène, (l'hémoglobine est alors sous forme oxygénée) mais également capable de relarguer facilement l'oxygène (l'hémoglobine est alors sous forme déoxygénée).

Cette propriété vis à vis de l'oxygène est caractérisée par la courbe (appelée courbe de Barcroft) qui correspond à la variation de la quantité d'oxygène retenue par unité de masse du transporteur en fonction de la pression partielle de l'oxygène contenue dans l'atmosphère à laquelle l'hémoglobine est exposée.

Dans le cas d'une solution aqueuse de l'hémoglobine native (concentration 15 µmoles/litre) cette variation est représentée par le tracé de référence représenté sur la figure 1, représenté à pH 7.2 à 25 °C.

L'un des paramètres associé à cette courbe est la pression de demi-saturation ($P_{50}$) qui est la pression partielle de l'oxygène à laquelle il faut soumettre la solution d'hémoglobine pour qu'elle absorbe une masse d'oxygène équivalant à 50 % de la quantité maximum à laquelle elle est capable de se combiner.Or dans le cas de l'hémoglobine modifiée (hémoglobine couplée à des polymères) comme indiqué ci-dessus, la courbe de la figure 1 représentant le pourcentage de l'oxygène combiné à l'hémoglobine sous des pressions partielles d'oxygène déterminées, subit une translation vers la gauche par rapport à la courbe correspondant à l'hémoglobine native.

Ceci signifie que la pression $P_{50}$ de l'hémoglobine modifiée est inférieure à celle de l'hémoglobine native; en d'autres termes l'hémoglobine modifiée présente une affinité trop forte vis à vis de l'oxygène, ce qui a pour inconvénient que l'oxygène ne peut être restituée rapidement aux tissus irrigués.

Cette modification des propriétés oxyphoriques de l'hémoglobine modifiée peut s'expliquer notamment pour les raisons suivantes :

- l'hémoglobine native subit à l'intérieur du globule rouge, l'influence du diphospho-2,3-glycérate (2,3 DPG) qui est l'effecteur naturel intraérythrocytaire, qui s'associe avec les amines du site allostérique de la déoxyhémoglobine, ce qui abaisse l'affinité de l'hémoglobine vis à vis de l'oxygène.

Des améliorations pour remédier à cet inconvénient ont été proposées en effectuant le couplage entre des polymères et de l'hémoglobine, en présence d'effecteurs temporaires (ou ligands) tels que le diphospho-2,3-glycérate ou l'inositol hexaphosphate et en absence totale d'oxygène (brevet français n° 83.145.45).

Ces substances polyphosphatées complexent fortement le site allostérique de la déoxyhémoglobine, stabilisant cette dernière, et protègent de cette façon les sites aminés qui sont essentiels pour le processus de transport de l'oxygène.

Cependant ces effecteurs sont éliminés soit au cours des étapes de purification des conjugués polymères de l'hémoglobine, soit dans le plasma, dans le cas où la purification n'a pas entraîné l'élimination des effecteurs, en raison des conditions dissociatives du plasma.

Pour remédier à cet inconvénient, on a proposé d'avoir recours à des effecteurs permanents, par

exemple en fixant du pyridoxal-5'-phosphate (Japan Kokai Tokkyo JP n° 59.104.323).

On a également proposé d'utiliser des ligands avec plusieurs groupes phosphates qui, après fixation sur l'hémoglobine, diminuent sensiblement son affinité pour l'oxygène.

Cependant, l'un des inconvénients présentés par ces composés est que leur obtention met en oeuvre plusieurs opérations sur l'hémoglobine et en tout cas plus de deux, ce qui provoque la formation de produits de dégradation, notamment de méthémoglobine.

On a également proposé, dans la demande de brevet européen n° 142,125, des complexes ioniques polymère-ligand/hémoglobine, obtenus d'une part à partir de composés d'addition entre des ligands et des polymères hydrosolubles, et, d'autre part à partir d'hémoglobine, la liaison entre les composés d'addition polymère-ligands et l'hémoglobine étant une liaison ionique.

Ces complexes sont capables de réduire l'affinité de l'hémoglobine vis-à-vis de l'oxygène.

Mais, l'association purement ionique entre l'hémoglobine et les composés d'addition polymère-ligand n'est solide que si l'hémoglobine se trouve sous forme déoxygénée, ce qui implique que lorsque l'hémoglobine se réoxygène, les composés d'addition polymère-ligand sont éjectés du site de liaison phosphatique de l'hémoglobine, et il n'y a plus d'interaction ionique forte entre les susdits composés d'addition et hémoglobine.

Si on injecte dans l'organisme de tels complexes ioniques, on peut s'attendre à ce que le composé d'addition polymère-ligand, qui n'est pas fortement et spécifiquement associé à l'hémoglobine , quitte rapidement l'organisme, surtout si sa masse moléculaire n'est pas très élevée et à ce que l'hémoglobine sorte du circuit vasculaire.

L'invention a pour but de remédier à ces inconvénients en proposant de nouveaux conjugués macromoléculaires d'hémoglobine, dont la taille est telle que leur diffusivité extravasculaire est limitée, voire annulée, qui sont protégés contre les conditions dissociatives du plasma, dont les propriétés oxyphoriques ne sont pas altérées, mais améliorées par rapport à celles de l'hémoglobine libre, et dont la préparation limite le nombre d'étapes réactionnelles sur l'hémoglobine.

Cet aspect a été atteint par les nouveaux conjugués macromoléculaires de l'invention.

L'un des aspects de l'invention est de fournir de nouveaux conjugués macromoléculaires physiologiquement compatibles, et susceptibles de fixer l'oxygène réversiblement.

L'un des autres aspects de l'invention est de fournir de nouveaux conjugués macromoléculaires susceptibles de restituer l'oxygène plus facilement que l'hémoglobine libre.

L'un des aspects de l'invention est de proposer de nouveaux conjugués macromoléculaires faciles à synthétiser à l'échelle industrielle.

L'un des autres aspects de l'invention est de fournir de nouveaux conjugués macromoléculaires d'hémoglobine obtenus en mettant en oeuvre un procédé dans lequel l'hémoglobine n'est soumise qu'au plus à deux étapes, et généralement à une seule étape.

L'un des autres aspects de l'invention est de proposer de nouvelles solutions aqueuses contenant de nouveaux conjugués macromoléculaires d'hémoglobine susceptibles d'être utilisées comme substitut de sang, notamment dans les interventions nécessitant des transfusions ou la perfusion d'organe.

L'un des autres aspects de l'invention est de fournir des solutions d'hémoglobine dont les propriétés oxyphoriques, révélées par une augmentation de la $P_{50}$ in vitro par rapport à l'hémoglobine libre, demeurent stables in vivo.

L'un des autres aspects de l'invention est de proposer de nouveaux composés macromoléculaires d'hémoglobine présentant à la fois une affinité modérée par rapport à l'oxygène, ainsi qu'un volume hydrodynamique élevé, entraînant au cours d'expériences transfusionnelles, l'augmentation de la persistance intravasculaire de l'hémoglobine par suppression de l'hémoglobinurie.

Les nouveaux conjugués macromoléculaires d'hémoglobine selon l'invention présentant pour l'oxygène une affinité inférieure à celle de l'hémoglobine libre, sont caractérisés en ce qu'ils sont constitués

- d'une part par de l'hémoglobine, laquelle peut passer de façon réversible de la forme déoxygénée à la forme oxygénée,
- d'autre part, par un polymère P hydrosoluble, non toxique, de préférence non antigénique, hémocompatible, de masse moléculaire d'environ 1 000 à environ 500 000, de préférence d'environ 1 000 à environ 100 000 comportant des groupes polaires, de préférence des groupes hydroxyles, carboxyles ou amines,
- lequel conjugué macromoléculaire comporte des sites Z fixés sur le polymère P et contenant au moins une charge négative portée par des groupes anioniques constitués par des sulfates, et/ou phosphates et/ou carboxylates,
- le polymère P relié à l'hémoglobine
  . d'une part par l'intermédiaire d'au moins une liaison ionique établie entre l'un au moins des sites Z

portés par le polymère et l'hémoglobine, et

. d'autre part par l'intermédiaire d'au moins une liaison covalente établie, soit entre le polymère et l'hémoglobine, soit entre l'un des sites Z portés par le polymère et l'hémoglobine,

- le nombre de liaisons covalentes entre le polymère et l'hémoglobine étant tel que le conjugué macromoléculaire a une masse moléculaire moyenne d'environ 70 000 à environ 1 000 000, de préférence d'environ 70 000 à environ 500 000,
- la relation entre la charge négative, le nombre de sites et le nombre de monomères étant la suivante :
  - lorsque chaque site contient un groupe anionique unique constitué d'un sulfate ou d'un phosphate, il y a au moins un site tous les dix monomères du polymère,
  - lorsque l'un au moins des sites contient au moins deux groupes anioniques, constitués de sulfates et/ou de phosphates, il y a au moins un site pour le polymère,
  - lorsque chaque site contient des charges négatives provenant de carboxylates, il faut au moins deux groupes carboxylates sur un même site Z, et au moins un site Z tous les cinq monomères,
  - lorsque l'un au moins des sites contient au moins trois charges négatives provenant de carboxylates, il y a au moins un site pour le polymère.

On a constaté qu'une condition nécessaire pour que les conjugués macromoléculaires de l'invention soient hydrosolubles, dénués de toxicité, de préférence non antigéniques, et hémocompatibles est que les polymères P, susceptibles d'entrer dans la constitution des sus-dits conjugués macromoléculaires soient hydrosolubles, dénués de toxicité, de préférence non antigéniques, et hémocompatibles.

On a constaté que la présence sur la chaîne du polymère de sites Z, porteurs de groupes anioniques qui jouent le rôle d'effecteurs permanents, augmente la pression partielle pour laquelle 50 % de l'hémoglobine en solution est oxygénée, sans que cela puisse être imputé à l'existence éventuelle de ligands libres.

Ces effecteurs permanents sont tels qu'ils permettent également à l'hémoglobine de passer réversiblement de la forme désoxygénée à la forme oxygénée, avec une stabilisation plus forte de la conformation de la molécule d'hémoglobine sous forme déoxygénée, ce qui entraîne une diminution de l'affinité de l'hémoglobine vis à vis de l'oxygène.

En d'autres termes, ces effecteurs permettent à l'hémoglobine de transporter réversiblement l'oxygène, et notamment de relarguer facilement l'oxygène dans les tissus qui sont irrigués.

La liaison covalente est telle qu'elle confère au conjugué macromoléculaire de l'invention une stabilité le rendant non biodégradable ou peu biodégradable en milieu plasmatique, pendant le temps durant lequel le conjugué macromoléculaire doit assurer des fonctions oxyphoriques, c'est-à-dire 2 à 3 jours, ce qui supprime la diffusion extrarénale et extravasculaire de l'hémoglobine.

Dans les conjugués macromoléculaires de l'invention, l'hémoglobine est reliée au polymère par au moins une liaison covalente mais au-delà d'un nombre critique de liaisons covalentes entre les polymères et l'hémoglobine, notamment quand il y a phénomène de réticulation intermoléculaire, ceci nuit aux propriétés de l'hémoglobine.

Ce nombre critique de liaisons covalentes correspond au fait que la masse moléculaire moyenne en poids des conjugués macromoléculaires de l'invention ne doit pas être supérieure à environ 1 000 000.

La répartition de la densité de la charge négative doit être suffisante pour pouvoir former une liaison ionique avec les amines du site allostérique de l'hémoglobine, c'est à dire le site de l'effecteur naturel intraérythrocytaire, le diphospho-2,3 glycérate.

On a constaté que statistiquement un groupe sulfate ou phosphate tous les dix monomères est nécessaire et suffisant, mais ce rapport peut être supérieur à 1/10.

On a également constaté que lorsque l'un au moins des sites contient au moins deux groupes anioniques constitués d'ions phosphates et/ou sulfates, un seul site suffit pour toute la chaîne polymère, ce qui n'exclut pas la possibilité d'avoir plusieurs sites sur la chaîne.

En ce qui concerne la densité de charge négative dans le cas des carboxylates, il faut qu'il y ait au moins deux ions carboxylates sur le même site, et un site comportant deux ions carboxylates est nécessaire tous les cinq monomères.

On a constaté que lorsqu'il y a au moins trois ions carboxylates, la densité de charge du site est telle qu'un site suffit pour la chaîne de polymère, ce qui n'exclut pas la possibilité d'avoir plusieurs sites sur la chaîne.

Les polymères qui entrent dans la constitution des conjugués macromoléculaires de l'invention qui sont dégradés dans le milieu plasmatique ont une masse moléculaire moyenne en poids d'environ 1 000 à 500 000.

Les polymères qui entrent dans la constitution des conjugués macromoléculaires de l'invention et qui ne sont pas dégradés dans l'organisme doivent avoir une masse moléculaire moyenne en poids égale ou inférieure à environ 10 000, car au-delà de cette valeur les polymères passent difficilement la barrière

rénale et s'accumulent donc dans l'organisme.

C'est notamment le cas des polyalkylène glycols, de la polyvinylpyrrolidone, du polyméthylacrylate ou de certains polysaccharides, qui n'étant pas biodégradables doivent présenter une masse moléculaires moyenne en poids égale ou inférieure à environ 10 000.

Les polymères qui entrent dans la constitution des conjugués macromoléculaires de l'invention doivent être utilisés dans une gamme de poids moléculaire dans laquelle ils sont de préférence non antigéniques.

C'est ainsi que dans le cas du dextrane, son poids moléculaire doit être inférieur à environ 70 000.

Selon un mode de réalisation avantageux de l'invention, les ponts salins entre les groupes $NH_3^+$ et les groupes $COO^-$ internes de l'hémoglobine sont intacts lorsque l'hémoglobine est sous forme désoxygénée.

On désigne par ponts salins, certaines liaisons intra-moléculaires qui se forment entre des ions $NH_3^+$ et des anions $COO^-$, lorsque l'hémoglobine est sous forme désoxygénée.

Les ponts salins dans les conjugués macromoléculaires de l'invention doivent de préférence être intacts, car si les ions qui participent à la formation de ces ponts salins, sont engagés dans d'autres liaisons, le passage de l'hémoglobine de la forme oxygénée à la forme désoxygénée se fait très difficilement et de manière incomplète.

L'expression "ponts salins intacts" signifie qu'au moins 50 % des ponts salins ne sont pas altérés, et qu'avantageusement 80 à 90 %, voire 100 %, des ponts salins ne sont pas altérés.

Les critères permettant de vérifier si les ponts salins sont intacts sont notamment les suivants :

a) Courbe d'affinité vis à vis de l'oxygène :

si la courbe d'affinité vis à vis de l'oxygène (courbe de Barcroft) est déplacée vers la droite par rapport à celle de l'hémoglobine libre, ceci implique que les ponts salins ne sont pas modifiés.

b) Coefficient de Hill (n) :

ce paramètre traduit l'allure sigmoïde de la courbe de Barcroft et reflète le caractère plus ou moins coopératif de la fixation de l'oxygène. La valeur de n permet d'apprécier la permanence du comportement allostérique de l'hémoglobine. Dans le cas de l'hémoglobine native, ce coefficient est compris entre 2,7 et 3,0.

c) Effet Bohr :

il consiste à déterminer le comportement oxyphorique de l'hémoglobine à différents pH, ce qui permet d'évaluer les perturbations consécutives aux différentes manipulations.

Selon un autre mode de réalisation avantageux les polymères de l'invention sont choisis parmi les polysaccharides, notamment les hydroxyalkylamidons dont le groupe alkyle comporte de 2 à 4 atomes de carbone, l'inuline, le dextrane et ses dérivés, notamment le dextrane aminé, l'alcool polyvinylique, la polyvinylpyrrolidone, le polyméthacrylate et ses dérivés, les polypeptides, les polyalkène glycols dans lesquels le groupe alkène comporte de 2 à 5 atomes de carbone, notamment le polyéthylène glycol et le polypropylène glycol.

Selon un autre mode de réalisation avantageux de l'invention, Z provient de $OSO_3H$.

Selon un autre mode de réalisation avantageux de l'invention, Z provient du pyridoxalsulfate, du disulfate d'épinéphrine, du trisulfate d'épinéphrine, du disul-fate de norépinéphrine, du trisulfate de norépinéphrine, du disulfate de phénolphtaleïne.

Selon un autre mode de réalisation avantageux de l'invention, Z provient de $OPO_3H_2$.

Selon un autre mode de réalisation avantageux de l'invention, Z provient du pyridoxalphosphate, de l'adénosine triphosphate, de la phosphotyrosine, de la phosphosérine, de l'inositolhéxaphosphate et ses dérivés, de l'inositol tri-, tétra-, pentaphosphate et ses dérivés.

Selon un autre mode de réalisation avantageux de l'invention, Z provient de

$O-CH(COOH)_2$, 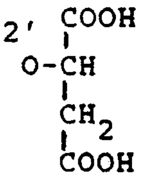, 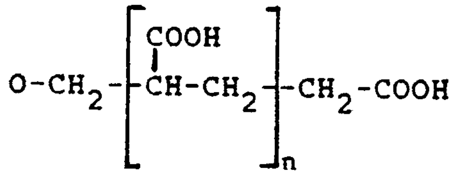,

n variant de 1 à 4.

Selon un autre mode de réalisation avantageux de l'invention, Z provient d'acide polycarboxylique contenant d'environ 2 à environ 10 atomes de carbone sur la chaîne principale, d'acide benzènecarboxylique comportant au moins deux fonctions carboxyliques, de diphospho 2,3-glycérate, d'acide citrique, du

propane 1, 2, 3 tricarboxylate ou du butane tétracarboxylate.

Selon un autre mode de réalisation avantageux de l'invention Z provient de l'hydroxy-2-formyl-5-phosphosérine benzamide de formule :

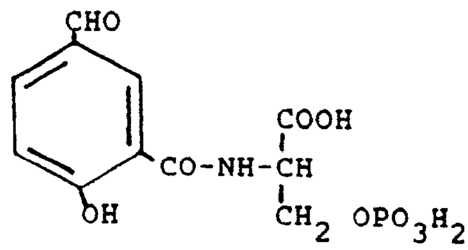

ou du formyl-4-phosphosérine benzamide de formule :

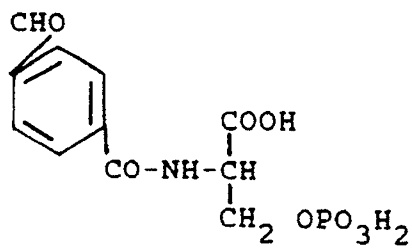

Selon un autre mode de réalisation avantageux de l'invention, le site Z comporte au moins trois groupes fonctionnels qui sont tels que

- l'une des fonctions est une fonction permettant d'attacher le groupe Z sur le polymère,
- l'une des fonctions est une fonction sulfate, phosphate ou carboxylate susceptible d'établir une liaison ionique avec une amine du site allostérique de l'hémoglobine,
- l'une des fonctions est une fonction aldéhyde, carboxylique ou OH, susceptible de former une liaison covalente avec un groupe $NH_2$ de l'hémoglobine.

La nature de la liaison qui relie Z au polymère dépend des éléments réactionnels en présence et des conditions de réaction.

Les liaisons qui unissent Z au polymère sont des liaisons éther, ester, amide ou amine.

A titre d'exemple, la liaison ester est obtenue par action de $H_2SO_4$ ou $H_3PO_4$ (après fixation $H_2SO_4$ donne le site $-OSO_3H$ et $H_3PO_4$ donne $-OPO_3H_2$) sur le polymère; la liaison éther est obtenue par greffage radicalaire de l'acide acrylique, ou par action d'acide chlorosuccinique, sur les groupes OH des polymères, la liaison amide est obtenue par réaction entre les groupes carboxylates des groupes Z et les groupes $NH_2$ des polymères; la liaison amine est obtenue par réaction entre les groupes aldéhydes des groupes Z et les groupes $NH_2$ des polymères, suivie d'une réduction. Cette dernière réaction peut également être effectuée simultanément à la première amination réductive.

Les liaisons covalentes entre le polymère et l'hémoglobine, sont établies entre les groupes $NH_2$ de l'hémoglobine et les groupes carboxyliques, aldéhydes ou hydroxyles qui se trouvent soit sur le polymère, soit sur les sites Z.

La liaison entre le polymère et l'hémoglobine est une liaison amide, imine, amine ou carbamate.

Selon un mode de réalisation de l'invention, les liaisons covalentes sont établies directement entre les groupes aldéhydes, carboxyles ou hydroxyles des polymères et les groupes $NH_2$ de l'hémoglobine.

Selon un autre mode de réalisation avantageux de l'invention, les liaisons covalentes sont établies entre les groupes aldéhydes, carboxyles ou hydroxyles des groupes Z fixés sur les polymères et les groupes $NH_2$ de l'hémoglobine.

Selon un mode de réalisation particulièrement avantageux de l'invention, les liaisons covalentes sont établies entre des groupes carboxyles et des groupes $NH_2$ de l'hémoglobine.

Des conjugués macromoléculaires d'hémoglobine avantageux selon l'invention sont ceux dans lesquels le polymère est constitué par du dextrane, de préférence de masse moléculaire moyenne en poids d'environ 40 000, contenant de préférence environ 5,4 moles de groupes $OSO_3H$ pour 10 moles de glucopyranose, les liaisons covalentes entre le polymère et l'hémoglobine résultant de la liaison entre des groupes aldéhydes préalablement formés sur le dextrane, de préférence à raison d'environ 18 groupements aldéhydes pour 100 unités glucosidiques et des groupes $NH_2$ de l'hémoglobine,

Des conjugués macromoléculaires d'hémoglobine avantageux selon l'invention sont ceux dans lesquels le polymère est constitué par du dextrane, de préférence de masse moléculaire moyenne en poids d'environ 10 000, contenant environ 9,8 moles de groupes $OSO_3H$ pour 10unités de glucopyranose, les liaisons covalentes entre le polymère et l'hémoglobine résultant de la liaison entre des groupes aldéhydes préalablement formés sur le dextrane, de préférence à raison d'environ 14 groupes aldéhydes pour 100 unités glucosidiques, et des groupes $NH_2$ de l'hémoglobine,

D'autres conjugués macromoléculaires d'hémoglobine avantageux selon l'invention sont ceux dans lesquels le polymère est constitué par du dextrane, de préférence de masse moléculaire moyenne en poids d'environ 6 000, contenant environ 1,7 ions sulfates pour 10 unités de glucopyranose, les liaisons covalentes entre le polymère et l'hémoglobine résultant de liaisons entre des groupes aldéhydes préalablement formés sur le dextrane, de préférence à raison d'environ 18 groupes aldéhydes pour 100 unités glucosidiques, et des groupes $NH_2$ de l'hémoglobine.

D'autres conjugués macromoléculaires d'hémoglobine avantageux selon l'invention sont ceux dans lesquels le polymère est constitué par du dextrane, de préférence de masse moléculaire moyenne en poids d'environ 40 000, contenant environ 12 moles de groupes $OPO_3H_2$ pour 10 moles de glucopyranose, les liaisons covalentes entre le polymère et l'hémoglobine résultant des liaisons entre des groupes aldéhydes préalablement formés sur le dextrane, de préférence à raison d'environ 18 groupes aldéhydes pour 100 unités glucosidiques, et des groupes $NH_2$ de l'hémoglobine.

D'autres conjugués macromoléculaires d'hémoglobine avantageux selon l'invention sont ceux dans lesquels le polymère est constitué par du dextrane aminé, de préférence de masse moléculaire moyenne en poids d'environ 40 000, les liaisons ioniques étant réalisées par l'intermédiaire de groupes carboxylates de l'acide benzène hexacarboxylique fixé sur le polymère et les liaisons covalentes étant réalisées par l'intermédiaire de groupes carboxyliques de l'acide hexacarboxylique non engagés dans les liaisons ioniques sus-définies et des groupes $NH_2$ de l'hémoglobine.

D'autres conjugués macromoléculaires d'hémoglobine avantageux selon l'invention sont ceux dans lesquels le polymère est constitué par du dextrane polycarboxylé, de préférence de masse moléculaire moyenne en poids d'environ 40 000, contenant en moyenne environ une chaîne de diacide carboxylique par monomère, les liaisons covalentes étant réalisées par l'intermédiaire de groupes carboxyliques non engagés dans la liaison ionique avec l'hémoglobine et des groupes $NH_2$ de l'hémoglobine.

Pour préparer les conjugués macromoléculaires d'hémoglobine de l'invention, on peut

- dans une première étape fixer les groupes Z sur le polymère
  * soit, en transformant préalablement le polymère P en polyol, si besoin est, puis en fixant les groupes Z par des méthodes connues en soi,
  * soit en utilisant un composé Z-Y dans lequel Y est une fonction active ou activable telle que aldéhyde, carboxylique, amine, hydroxyle, halogène, par exemple chlore, à l'aide d'un réactif approprié,
  * soit en effectuant un greffage radiochimique des groupes Z sur le polymère P,
- puis dans une deuxième étape, on fait réagir le polymère P comportant des groupes Z sur l'hémoglobine dans des conditions telles que l'hémoglobine ne subisse pas de dénaturation, en milieu aqueux de pH compris d'environ 5 à environ 9, pour former simultanément des liaisons covalentes et des liaisons ioniques entre le polymère et l'hémoglobine.

La première étape de préparation des conjugués macromoléculaires de l'invention et qui consiste à fixer des groupes Z sur le polymère, peut être effectuée par des méthodes connues, par exemple sulfatation, phosphorylation ou carboxyméthylation du polymère P sous forme de polyol. Dans le cas où le polymère P n'est pas un polyol, il est nécessaire de le transformer préalablement en polyol.

La première étape de préparation des conjugués macromoléculaires de l'invention peut également être effectuée en utilisant des composés du type Z-Y dans lesquels Y est une fonction aldéhyde, carboxylique, amine, hydroxyle ou halogène. Ces composés Z-Y peuvent être utilisés en ayant recours à des techniques chimiques classiques ; on peut par exemple fixer de l'acide penta ou héxacarboxylique, de l'acide diphosphoro-2,3 glycérique, du pyridoxal-5'-phosphate sur un polymère préalablement polyaminé.

Dans ce cas, il est impératif d'éliminer toute trace du composé Z-Y, par exemple par dessalage sur une colonne de filtration sur gel. En effet, il faut éviter la présence de composé Z-Y qui n'aurait pas réagi et qui, même en faible concentration avec les conjugués macromoléculaires de l'invention peut fausser les conclusions relatives aux propriétés des conjugués de l'invention.

On peut également avoir recours à toute autre méthode permettant de fixer des chaînes polyanioniques sur un polymère, tel qu'une méthode de greffage radiochimique.

La seconde étape mentionnée ci-dessus est une étape de réaction entre le polymère et l'hémoglobine et peut, si nécessaire, être éventuellement précédée d'une phase d'activation du polymère avant que le

polymère ne soit mis à réagir avec l'hémoglobine; mais cette activation peut être pratiquement simultanée avec la réaction entre le polymère et l'hémoglobine.

Au cours de la seconde étape, le polymère réagit avec l'hémoglobine et d'une part les liaisons ioniques entre les sites Z du polymère et l'hémoglobine et d'autre part les liaisons covalentes entre le polymère et l'hémoglobine se forment.

Dans cette seconde étape,

* soit les fonctions carboxyle, aldéhyde ou hydroxyle des sites Z, sont mises en jeu,
* soit les fonctions carboxyle, aldéhyde ou hydroxyle du polymère sont mises en jeu.

Dans le cas où ce sont des groupes carboxyles qui sont mise en jeu avec des groupes $NH_2$ de l'hémoglobine, on peut utiliser pour activer les groupes carboxyles les réactifs classiquement utilisés dans la synthèse peptidique, tels que les carbodiimides hydrosolubles, notamment le chlorhydrate de N'-éthyl-N-(3-diméthyl-aminopropyl)carbodiimide (EDCI), la N.hydroxysuccinimide, ou l'éthoxy-2-quinoline-1-carboxylate d'éthyle (EEDQ).

La liaison obtenue est alors une liaison amide.

Dans le cas où ce sont les groupes aldéhyde qui sont mis en jeu avec des groupes $NH_2$ de l'hémoglobine, on peut par exemple avoir recours à l'amination réductive.

L'amination réductive d'un aldéhyde consiste à former une imine par l'action d'une amine, et à réduire simultanément l'imine en amine à l'aide d'un réducteur tel que $NaBH_4$, $NaCNBH_3$, le diméthylaminoborane ou HCOOH.

La liaison obtenue est alors une liaison amine.

Dans le cas où ce sont des groupes aldéhydes qui sont mis en jeu avec des groupes $NH_2$ de l'hémoglobine, on peut également opérer dans des conditions telles que la liaison obtenue soit une liaison imine, laquelle doit ensuite être stabilisée en amine par réduction avec un réducteur doux, tel que l'un de ceux mentionnés ci-dessus.

Lorsque ni les groupes Z, ni le polymère P ne comportent de groupes aldéhydes, ou de groupes carboxyliques, mais qu'ils renferment des groupes hydroxyles dont certains sont sur des carbones adjacents, on peut former des groupes aldéhydes, sur le polymère P par exemple par oxydation périodique de ce dernier, notamment à l'aide de $NaIO_4$.

La liaison obtenue entre ces groupes aldéhydes et les amines de l'hémoglobine est alors une liaison imine, laquelle doit être stabilisée en amine par réduction, par exemple avec $NaBH_4$.

Lorsque ni les sites Z, ni le polymère P ne comportent de groupes aldéhydes ou carboxyliques, le polymère comportant des groupes OH peut être mis en jeu sur des groupes $NH_2$ de l'hémoglobine à l'aide d'un réactif approprié tel que le bromure de cyanogène ou le carbonyldiimidazole. Dans ce cas, la liaison obtenue est une liaison carbamate

$$-O-\overset{\overset{\displaystyle O}{\|}}{C}-NH-$$

Les conjugués macromoléculaires d'hémoglobine dans lesquels les liaisons covalentes entre le polymère sont des liaisons imines ne sont pas stables dans l'organisme, c'est pourquoi il convient de stabiliser la fonction imine en amine, par exemple par réduction par $NaBH_4$, $NaCNBH_3$, ou le diméthylaminoborane.

Les conjugués macromoléculaires d'hémoglobine dans lesquels les liaisons covalentes sont des liaisons imines sont des composés intermédiaires nouveaux, qui peuvent être utilisés comme réactifs, notamment in vitro.

Dans le cas où il est nécessaire de stabiliser les liaisons covalentes, l'hémoglobine n'est en tout cas pas soumise à plus de deux étapes de réaction, et dans les autres cas, qu'il y ait activation ou non du polymère P, l'hémoglobine est soumise à une seule étape de réaction, ce qui est particulièrement avantageux car le nombre limité d'étapes de réaction sur l'hémoglobine (au plus égal à deux) est l'une des conditions essentielles pour que, en plus du rendement de la réaction, l'hémoglobine ne soit pas dénaturée.

Au cours de la deuxième étape, il est en effet essentiel que l'hémoglobine ne subisse au cours de la formation du conjugué macromoléculaire de l'invention ni dénaturation importante, ni réduction sensible de la mobilité relative des diverses entités qui la constituent afin de conserver, au moins en partie, ses propriétés oxyphoriques.

La deuxième étape de réaction a lieu en milieu aqueux, tamponné ou non, à un pH d'environ 5 à environ 9, éventuellement en présence d'un activateur comme indiqué ci-dessus, en l'absence d'oxygène, pendant une durée suffisante et de préférence inférieure à celle à partir de laquelle il y a apparition de formation importante de methémoglobine (supérieure à environ 5 %), à une température assurant une

conservation correcte de l'hémoglobine.

Le milieu aqueux est tamponné par des tampons classiquement utilisés, pour stabiliser le pH à la valeur souhaitée.

La durée de réaction est d'environ 30 mn à environ 20 h, et avantageusement d'environ 1 h à environ 8 h, à une température d'environ 3 à environ 30 °C.

La durée de la réaction dépend notamment de la température à laquelle on travaille.

L'hémoglobine utilisée est sous forme désoxygénée, et on utilise avantageusement une solution d'hémo-globine à 9 %.

Le rapport des concentrations molaires entre le polymère et l'hémoglobine doit en outre être tel que la plus grande partie des molécules d'hémoglobine soit liée de façon covalente avec le polymère polyanionique.

A titre d'exemple, on peut indiquer qu'en faisant réagir, à une température d'environ 3 à 30 °C, en solution dans un milieu aqueux à pH voisin de 6,5, un mélange de polymère dextrane, dont la masse moléculaire moyenne en poids est d'environ 10 000 à environ 40 000, et d'hémoglobine dans lequel le rapport des concentrations molaires entre le dextrane et l'hémoglobine est d'environ 0,5 à environ 2, on obtient des conjugués macromoléculaires de l'invention, dont l'examen chromatographique, par exemple par perméation sur gel montre qu'il n'y a plus d'hémoglobine libre.

Les conjugués macromoléculaires de l'invention présentent l'avantage d'être synthétisés facilement, sans formation ou presque de methémoglobine, (inférieure à environ 5 %) et de n'impliquer qu'un nombre limité de réactions sur l'hémoglobine, ce qui évite sa dénaturation.

Par ailleurs, les conjugués macromoléculaires de l'invention ne renferment pas d'effecteurs de petite masse moléculaire, non liés, qui entraînent dans les expériences in vitro une augmentation de la $P_{50}$, mais qui peuvent être facilement éliminés par diffusion extrarénale ou extravasculaire au cours des essais in vivo, et dont l'effet s'annule rapidement.

Les conjugués macromoléculaires de l'invention présentent l'avantage de n'avoir pour l'oxygène qu'une affinité modérée, et d'avoir un volume hydrodynamique élevé, ce qui pendant les opérations transfusionnelles augmente la persistance intravasculaire de l'hémoglobine par suppression de l'hémoglobinurie.

Les conjugués d'hémoglobine liés irréversiblement à des polymères polyanioniques préparés selon l'invention peuvent, lorsqu'ils sont dissous dans des solutions aqueuses de composition convenable, jouer le rôle de substituts de sang, notamment dans des interventions nécessitant des transfusions ou la perfusion d'organe.

L'invention vise par conséquent, également les solutions aqueuses renfermant les conjugués décrits ci-dessus et notamment les solutions rendues isotoniques du sang, par dialyse prolongée contre une solution de Tyrode ( de composition NaCl 8 g/l ; KCl 0,2 g/l ; $CaCl_2$ 0,2 g/l ; $MgCl_2$ 0,1 g/l ; $NaH_2PO_4$ 0,05 g/l ; $NaHCO_3$ 1 g/l ; D.glucose 1 g/l) par exemple et concentration par ultrafiltration jusqu'à obtenir pour l'hémoglobine, une concentration d'environ 7 %.

Des préparations de conjugués macromoléculaires d'hémoglobine obtenus selon l'invention, ont été examinées en tant que transporteurs d'oxygène potentiels. On a pu montrer qu'elles sont effectivement capables de fixer l'oxygène réversiblement et en particulier de le restituer plus facilement que l'hémoglobine libre ainsi que l'illustrent les courbes d'affinité pour l'oxygène des produits décrits dans les exemples 1, 3 et 5 ci-après et représentés sur la figure 5 qui sera commentée ci-dessous. Il apparaît en effet, que ces préparations sont caractérisées par des pressions de demi-saturation ($P_{50}$) qui peuvent être très élevées (de 900 à 5 000 Pa) alors que dans les mêmes conditions (NaCl 0.05 M, pH 7, 25 °C) celle de l'hémoglobine native est égale à environ 480 Pa.

Ainsi ces composés peuvent être utilisés pour fournir à des tissus ischémiés des quantités d'oxygène importantes. Ils peuvent également être utilisés en transfusion et être administrés à des patients, sous forme d'une solution aqueuse rendue isotonique du sang, en présence ou non d'excipients. Les composés peuvent aussi être lyophilisés en présence ou non d'un cryo-protecteur ou atomisés, et être redissous dans l'eau avant utilisation.

L'invention sera mieux comprise à l'aide des exemples qui suivent ci-après donnés à titre non limitatif.

EXEMPLE 1 :
Synthèse d'un conjugué covalent d'hémoglobine et de sulfate de dextrane (masse moléculaire du dextrane : 40 000)

On prépare du sulfate sodique de dextrane contenant 8 % de soufre (soit environ 5,4 moles d'ions sulfates pour 10 moles de glucopyranose) à partir de dextrane dont la masse moléculaire moyenne en poids est de 40 000 (Pharmacia, Uppsala, Suède), comme indiqué dans C.R. RICKETTS. Biochem. J. 1952,

51, 129.

L'activation de ce sulfate de dextrane est réalisée par oxydation périodique comme indiqué dans E. DELLACHERIE et al., Biochem. Biophys. Acta 1983, 749, 106. La quantité de $NaIO_4$ utilisée est telle que le sulfate de dextrane dialdéhyde contient environ 18 groupements aldéhydiques pour 100 unités glucopyranoses.

Ce composé est dialysé longuement contre de l'eau, puis lyophilisé et gardé au froid, sous vide.

On dissout 10 g de ce sulfate de dextrane dialdéhyde dans 100 ml d'une solution de NaCl à 20% et la solution est désoxygénée on boîte à gants, mise sous vide, puis on effectue un balayage de l'azote, cette opération étant répétée 3 fois. On ajoute 120 ml d'une solution d'hémoglobine à 9%, désoxygénée par le même procédé et on ajuste le pH du mélange résultant à 7,5 par additon de soude 0,1N désoxygénée.

On maintient la solution à 25° C sans agitation et on poursuit la réaction durant 6 heures.

On ajoute alors 3,5 ml d'une solution extemporanée de 200 mg de $NaBH_4$ dans 5 ml de NaOH $10^{-3}$N. On laisse la réaction se poursuivre pendant 1 h puis on abaisse le pH à 7 pour détruire l'excès de réducteur.

L'examen du chromatogramme obtenu en filtration sur gel sur une colonne de type TSK. SW 3000 Beckman (zone d'exclusion 300 000 daltons) montre qu'il reste très peu d'hémoglobine non liée. On peut se reporter à la figure 2, sur laquelle on a représenté la variation de la densité optique en fonction du volume d'élution, et qui montre que le pic correspondant à l'hémoglobine non liée est de très faible amplitude.

La courbe d'affinité pour l'oxygène de ce conjugué de l'invention est représentée sur la figure 5.

La $P_{50}$ est de 850 Pa. (25° C, NaCl 0,05 M, pH 7)

Le taux de méthémoglobine est de 5 %. On mesure ce taux comme indiqué dans EVELYN K.A. et MALLOY H.I., J. Biol. Chem. 1938, 126, 655.

EXEMPLE 2

Synthèse d'un conjugué covalent d'hémoglobine et de sulfate de dextrane.
(Masse moléculaire du dextrane = 10 000)

1) Préparation du susdit conjugué selon l'invention :

On réalise la réaction entre de la déoxyhémoglobine et du sulfate sodique de dextrane dialdéhyde préparé selon la méthode décrite dans l'exemple 1 à partir de dextrane dont le poids moléculaire est de 10 000, et contenant 12 % de soufre (soit 9,8 ions sulfates pour 10 unités glucopyranoses) et 14 groupes aldéhydiques pour 100 unités glucosidiques.

Pendant 7 heures à 25° C et en l'absence totale d'oxygène,on fait réagir 10 g de sulfate de dextrane dialdéhyde et 120 ml d'une solution de déoxyhémoglobine à 9 % (mêmes conditions que pour la réaction décrite dans l'exemple 1), et toute l'hémoglobine est liée au polymère et la $P_{50}$ du conjugué est de 1060 Pa (25° C, NaCl 0,05 M, pH 7).

2) Comparaison entre un mélange d'hémoglobine et de sulfate de dextrane et d'un conjugué covalent selon l'invention d'hémoglobine et de sulfate de dextrane préparé au 1) :

On a vérifié par chromatographie d'exclusion stérique qu'un mélange de sulfate de dextrane (très bon effecteur macromoléculaire de l'hémoglobine et qui s'associe donc très fortement avec la déoxyhémoglobine) et d'hémoglobine oxygénée conduit à la sortie de colonne de chromatographie, à deux pics séparés correspondant respectivement à de l'hémoglobine libre et à du sulfate de dextrane libre.

On peut se reporter à la figure 6 sur laquelle on a représenté la variation de l'indice de réfraction en fonction du volume d'élution résultant de l'injection d'un mélange sulfate de dextrane/hémoglobine. La courbe obtenue est représentée en traits pleins et elle comporte deux pics dont le premier (au volume d'élution d'environ 60 ml) correspond à de l'hémoglobine libre et le deuxième (au volume d'élution d'environ 90 ml) correspond à du sulfate de dextrane libre.

La figure 6 comporte également la courbe en traits pointillés correspondant à l'injection du conjugué covalent sulfate de dextrane/hémoglobine conformément à l'invention, dont la synthèse a été indiquée ci-dessus au 1).

Cette courbe ne comporte qu'un seul pic d'élution correspondant à une masse moléculaire supérieure respectivement à celle de l'hémoglobine libre et à celle du sulfate de dextrane libre.

La séparation permettant d'obtenir ces deux courbes a été réalisée sur une colonne Ultrogel AcA 54 IBF (zone d'exclusion 90 000) de 55 cm, dans les conditions suivantes : Tampon Tris 0,05 M ; NaCl 0,15 M

; pH 7,2 ; débit 30 ml/h.

On en déduit qu'il est nécessaire de fixer de manière irréversible le polymère-ligand sur l'hémoglobine de façon à ce que :

a) les groupes effecteurs du polymère restent toujours à proximité de l'hémoglobine de sorte qu'ils puissent jouer leur rôle de modulateurs vis-à-vis de l'hémoglobine dès que celle-ci libère son oxygène ;

b) le polymère et l'hémoglobine en restant associés l'un à l'autre, puissent être maintenus dans le circuit vasculaire.

On constate également que l'affinité pour $O_2$ de l'hémoglobine ainsi fixée de façon covalente, n'est pas augmentée par rapport à celle de l'hémoglobine libre et même dans certains cas - tout particulièrement lorsque l'on utilise comme polymères effecteurs des polycarboxylates de dextrane (dextrane benzène hexacarboxylate, dextrane de polyacrylate) - on a remarqué que la $P_{50}$ de l'hémoglobine après couplage avec le polymère pouvait être supérieure à celle de l'hémoglobine libre en présence du même polymère avant le couplage covalent.

On en déduit donc que les conjugués de l'invention dans lesquels les polymères sont fixés de manière covalente, donc irréversible, sur la molécule d'hémoglobine sont tels que l'hémoglobine après l'immobilisation présente une meilleure activité fonctionnelle, car pendant la réaction chimique, l'hémoglobine est sous forme déoxygénée et dans cette forme, certaines fonctions chimiques sont masquées, ce qui évite ainsi qu'elles soient touchées par le couplage.

EXEMPLE 3 :

Synthèse d'un conjugué covalent d'hémoglobine et de sulfate de dextrane. (Masse moléculaire du dextrane = 6000).

On réalise la réaction entre de la déoxyhémoglobine et du sulfate sodique de dextrane dialdéhyde préparé selon la méthode décrite dans l'exemple 1 à partir de dextrane dont la masse moléculaire est de 6000, et contenant 3 % de soufre (soit 1,7 ions sulfates pour 10 unités glucopyranoses) et 18 groupes aldéhydiques pour 100 unités glucosidiques. Pendant 6 heures à 25° C et on l'absence totale d'oxygène on fait réagir 10 g de sulfate de dextrane dialdéhyde et 120 ml d'une solution de déoxyhémoglobine à 9 % (mêmes conditions que pour la réaction décrite dans l'exemple 1), et toute l'hémoglobine est liée au polymère et la $P_{50}$ du conjugué est de 602 Pa (25° C, NaCl 0,05 M, pH7).

EXEMPLE 4 :

Synthèse d'un conjugué covalent d'hémoglobine et de phosphate de dextrane.
(masse moléculaire du dextrane = 40 000)

On prépare du phosphate sodique de dextrane contenant 12 % de phosphore (soit environ 12 moles d'ions phosphates pour 10 moles de glucopyranose) à partir de dextrane de poids moléculaire = 40 000 comme indiqué dans G. DAUL et al, Industrial and Engineering Chemistry, 1954, 46, 1042.

On réalise l'activation de ce phosphate de dextrane par oxydation périodique comme cela est décrit dans l'exemple 1 de façon à créer environ 18 groupements aldéhydiques pour 100 unités glucosidiques.

On met à réagir ce phosphate de dextrane dialdéhyde avec la déoxyhémoglobine dans des proportions et des conditions tout à fait identiques à celles décrites dans l'exemple 1. Après 6 heures de réaction, puis réduction par $NaBH_4$ comme indiqué à l'exemple 1, toute l'hémoglobine est liée. La courbe d'affinité pour l'oxygène de ce conjugué est représentée à la figure 5.

La $P_{50}$ du conjugué est de 1240 Pa (25° C, NaCl 0,05 M, pH 7). Le taux de méthémoglobine est de l'ordre de 4 %.

EXEMPLE 5

Synthèse d'un conjugué covalent d'hémoglobine et de polycarboxylate de dextrane.
(masse moléculaire du dextrane = 40 000)

(1ère méthode)

Du dextrane aminé contenant $5.10^{-4}$ moles de $NH_2$ par g de produit sec (soit 8 moles de $NH_2$ pour 100 moles de glucopyranose) est préparé selon P. HUBERT et al, Proc. Natl. Acad. Sci. USA 1978, 75, 3143, par action de l'ammoniaque sur du dextrane activé à l'épichlorhydrine.

1 g de ce dextrane aminé est dissous dans 20 ml d'eau et le pH est ajusté à 6,5 avec HCl 0.1N. On ajoute ensuite 1,7 g d'acide benzène hexacarboxylique, puis 1 g de chlorhydrate de N'-éthyl-N-(3-diméthyl-aminopropyl)carbodiimide (EDCI). Le pH est ramené à 6,5 et la réaction est poursuivie pendant 3 jours à 20° C. Après dialyse contre une solution d'acétate de sodium 0,5 M, le mélange est traité à l'anhydride acétique pour bloquer les fonctions amines non substituées du dextrane. La solution est alors épurée des contaminants de petite masse moléculaire par dessalage sur une colonne d'Ultrogel AcA 202 (IBF-France) avec un tampon phosphate 0,2 M, pH 7,2.

Après une dialyse prolongée contre de l'eau, la solution contenant le polymère polyanionique est lyophilisée. Le composé est conservé au froid sous vide.

0,5 g de ce polycarboxylate de dextrane sont dissous dans 25 ml de NaCl aqueux 0,05 M. On amène le pH à 6,5 avec de la soude 0,1 N et on désoxygène la solution comme il est indiqué dans l'exemple 1. On ajoute alors 15 ml d'une solution d'hémoglobine à 10 %, désoxygénée dans les mêmes conditions. Au mélange maintenu en boîte à gants, sous balayage d'azote, on ajoute ensuite 60 mg d'EDCI et la réaction est poursuivie pendant 10 heures à 20° C. On vérifie qu'il n'y a plus d'hémoglobine libre sur le chromatogramme obtenu sur une colonne d'Ultrogel AcA 34. (IBF-France). On peut se reporter à la figure 3, sur laquelle on a représenté la variation de la densité optique en fonction du volume d'élution, et qui montre que le pic correspondant à l'hémoglobine non liée est de faible amplitude. La $P_{50}$ du conjugué est de 1360 Pa (25° C, NaCl 0,05 M, pH 7).

EXEMPLE 6 :

Synthèse d'un conjugué covalent d'hémoglobine et de polycarboxylate de dextrane.
(masse moléculaire du dextrane : 40 000)

2ème méthode :

Du polycarboxylate de dextrane, contenant des chaînes oligomères d'acide polyacrylique, est préparé en créant le long de la chaîne polysaccharidique des radicaux sur lesquels est initiée en milieu aqueux l'oligomérisation d'acide acrylique. Ce polycarboxylate sodique de dextrane est purifié par dialyse prolongée contre de l'eau, puis lyophilisé.

1 g de ce polycarboxylate sodique de dextrane contenant en moyenne un chaînon de diacide carboxylique par unité glucosidique, est mis à réagir avec la déoxyhémoglobine dans des conditions tout à fait identiques à celles qui sont décrites dans l'exemple 5.

La réaction est arrêtée au bout de 4 heures et le mélange est chromatographié sur une colonne Ultrogel AcA 34 (IBF-France) ce qui permet de vérifier qu'il n'y a plus d'hémoglobine libre. On peut se reporter à la figure 4, sur laquelle on a représenté la variation de la densité optique en fonction du volume d'élution, et qui montre que le pic correspondant à l'hémoglobine non liée est de faible amplitude.

La courbe d'affinité pour l'oxygène de ce conjugué est représentée sur la figure 5. La $P_{50}$ est de 5500 Pa. (25° C, NaCl 0,05 M, pH7).

Essais de toxicité aiguë sur animaux :

Les différents polymères polyanioniques décrits dans les exemples ont été injectés à des souris de souche SWISS dans les conditions suivantes : les polymères sont dissous dans de l'eau distillée à une concentration comprise entre 2,5 g et 5 g/l et le pH est ajusté à 7,4. 0,5 ml de chaque solution sont alors injectés par voie intrapéritonéale à 5 souris, dont le comportement est ensuite observé pendant une période de 7 jours. Ces essais n'ont permis de déceler aucun symptôme de toxicité aiguë.

**Revendications**

**Revendications pour les Etats contractants suivants : BE, CH, DE, ES, FR, GB, GR, IT, LI, LU, NL, SE**

**1.** Conjugué macromoléculaire hydrosoluble d'hémoglobine, non biodégradable ou peu biodégradable, pendant le temps durant lequel le conjugué macromoléculaire doit assurer des fonctions oxyphoriques dans le plasma, présentant pour l'oxygène une affinité inférieure à celle de l'hémoglobine libre, caractérisé en ce qu'il est constitué :
- d'une part par de l'hémoglobine, laquelle peut passer de façon réversible de la forme déoxygénée à la forme oxygénée,
- d'autre part par un polymère P hydrosoluble, non toxique, de préférence non antigénique, hémocompatible, de masse moléculaire d'environ 1 000 à environ 500 000, de préférence d'environ 1 000 à environ 100 000, comportant des groupes polaires, de préférence des groupes hydroxyles, carboxyles ou amines,

- lequel conjugué macromoléculaire comporte des sites Z attachés sur le polymère P et contenant au moins une charge négative portée par des groupes anioniques constitués par des sulfates et/ou phosphates et/ou carboxylates,
- le polymère P étant relié à l'hémoglobine
  . d'une part par l'intermédiaire d'au moins une liaison ionique établie entre l'un au moins des sites Z portés par le polymère et l'hémoglobine, et
  . d'autre part par l'intermédiaire d'au moins une liaison covalente établie, soit entre le polymère et l'hémoglobine, soit entre l'un au moins des sites Z portés par le polymère et l'hémoglobine,
- le nombre de liaisons covalentes entre le polymère et l'hémoglobine étant tel que le conjugué macromoléculaire a une masse moléculaire moyenne d'environ 70 000 à environ 1 000 000, de préférence d'environ 70 000 à environ 500 000,
- la relation entre la charge négative, le nombre de sites et le nombre de monomères étant la suivante :
  . lorsque chaque site contient un groupe anionique unique constitué d'un sulfate ou d'un phosphate, il y a au moins un site tous les dix monomères du polymère,
  . lorsque l'un au moins des sites contient au moins deux groupes anioniques, constitués de sulfates et/ou de phosphates, il y a au moins un site pour le polymère,
  . lorsque chaque site contient des charges négatives provenant de carboxylates, il faut au moins deux groupes carboxylates sur un même site Z, et au moins un site Z tous les cinq monomères,
  . lorsque l'un au moins des sites contient au moins trois charges négatives provenant de carboxylates, il y a au moins un site pour le polymère.

2. Conjugué macromoléculaire selon la revendication 1, caractérisé en ce que les ponts salins entre les groupes $NH_3^+$ et les groupes $COO^-$ internes de l'hémoglobine sont intacts lorsque l'hémoglobine est sous forme déoxygénée,

3. Conjugué macromoléculaire selon la revendication 1, caractérisé en ce que le polymère P est choisi parmi les polysaccharides, notamment les hydroxyalkylamidons dont le radical alkyle comporte de 2 à 4 atomes de carbone, l'inuline, le dextrane et ses dérivés, notamment le dextrane aminé, l'alcool polyvinylique, la polyvinylpyrrolidone, le polyméthacrylate et ses dérivés, les polypeptides, les polyalkène glycols dans lesquels le groupe alkène comporte de 2 à 5 atomes de carbone, notamment le polyéthylène glycol et le polypropylène glycol.

4. Conjugué macromoléculaire selon la revendication 1, caractérisé en ce que le polymère P a une masse moléculaire moyenne inférieure ou égale à 70 000, lorsqu'il est constitué par le dextrane et ses dérivés, et une masse moléculaire inférieure ou égale à 10 000 lorsqu'il est choisi parmi les polyalkylène glycols, la polyvinylpyrrolidone ou le polyméthylacrylate.

5. Conjugué macromoléculaire selon la revendication 1, caractérisé en ce que le site Z est relié au polymère par l'intermédiaire d'une fonction ester, éther, amide ou amine.

6. Conjugué macromoléculaire selon la revendication 1, caractérisé en ce que le site Z provient de

$$OSO_3H,\ OPO_3H_2,\ O\text{-}CH(COOH)_2,\ \text{-}O\text{-}CH(COOH)\text{-}CH_2\text{-}COOH,$$

$$\text{-}O\text{-}CH_2\text{-}\left[\begin{matrix}(CH\text{-}CH_2)\\ |\\ COOH\end{matrix}\right]_n\text{-}CH_2\text{-}COOH,$$

n variant de 1 à environ 4,
ou provient du pyridoxalsulfate, du pyridoxalphosphate, de l'adénosine triphosphate, de la phosphotyrosine, de la phosphosérine, de l'inositolhéxaphosphate et ses dérivés, d'acide polycarboxylique contenant de 2 à 10 atomes de carbone sur la chaîne principale, de l'acide benzènecarboxylique comportant au moins trois fonctions carboxyliques, du 2,3 diphosphoglycérate.

7. Conjugué macromoléculaire selon la revendication 1, caractérisé en ce que l'hémoglobine est liée de façon covalente au polymère par l'intermédiaire de liaisons établies d'une part entre des groupes

aldéhydes, des groupes carboxyles, ou des groupes OH et d'autre part des groupes $NH_2$ de l'hémoglobine.

8. Conjugué macromoléculaire selon la revendication 1, caractérisé en ce que les liaisons covalentes entre le polymère et l'hémoglobine sont établies directement entre des groupes aldéhydes, des groupes carboxyles ou des groupes OH portés par le polymère et des groupes $NH_2$ de l'hémoglobine, ou à partir des groupes aldéhydes, carboxyles ou hydroxyles provenant des sites Z fixés sur le polymère et des groupes $NH_2$ de l'hémoglobine.

9. Conjugué macromoléculaire selon la revendication 1, caractérisé en ce que le site Z comporte au moins trois groupes fonctionnels qui sont tels que
   - l'une des fonctions est une fonction permettant de fixer le site Z sur le polymère,
   - l'une des fonctions est une fonction sulfate, phosphate ou carboxylate susceptible d'établir une liaison ionique avec une amine du site allostérique de l'hémoglobine,
   - l'une des fonctions est une fonction aldéhyde, carboxyle ou OH susceptible de former une liaison covalente avec un groupe $NH_2$ de l'hémoglobine.

10. Conjugué macromoléculaire selon la revendication 1, caractérisé en ce que le polymère est constitué par du dextrane, de préférence de masse moléculaire moyenne en poids d'environ 40 000, contenant de préférence environ 5,4 moles de groupes $OSO_3H$ pour 10 moles de glucopyranose, les liaisons covalentes entre le polymère et l'hémoglobine résultant de la liaison entre des groupes aldéhydes préalablement formés sur le dextrane, de préférence à raison d'environ 18 groupements aldéhydes pour 100 unités glucosidiques et des groupes $NH_2$ de l'hémoglobine.

11. Conjugué macromoléculaire selon la revendication 1, caractérisé en ce que le polymère est constitué par du dextrane, de préférence de masse moléculaire moyenne en poids d'environ 10 000, contenant environ 9,8 moles de groupes $OSO_3H$ pour 10 unités de glucopyranose, les liaisons covalentes entre le polymère et l'hémoglobine résultant de la liaison entre des groupes aldéhydes préalablement formés sur le dextrane, de préférence à raison d'environ 14 groupes aldéhydes pour 100 unités glucosidiques, et des groupes $NH_2$ de l'hémoglobine.

12. Conjugué macromoléculaire selon la revendication 1, caractérisé en ce que le polymère est constitué par du dextrane, de préférence de masse moléculaire moyenne en poids d'environ 6 000, contenant environ 1,7 ions sulfates de groupes $OSO_3H$ pour 10 unités de glucopyranose, les liaisons covalentes entre le polymère et l'hémoglobine résultant de la liaison entre des groupes aldéhydes préalablement formés sur le dextrane, de préférence à raison d'environ 18 groupes aldéhydes pour 100 unités glucosidiques, et des groupes $NH_2$ de l'hémoglobine.

13. Conjugué macromoléculaire selon la revendication 1, caractérisé en ce que le polymère est constitué par du dextrane, de préférence de masse moléculaire moyenne en poids d'environ 40 000, contenant environ 12 moles de groupes $OPO_3H_2$ pour 10 moles de glucopyranose, les liaisons covalentes entre le polymère et l'hémoglobine résultant des liaisons entre des groupes aldéhydes préalablement formés sur le dextrane, de préférence à raison d'environ 18 groupes aldéhydes pour 100 unités glucosidiques, et des groupes $NH_2$ de l'hémoglobine.

14. Conjugué macromoléculaire selon la revendication 1, caractérisé en ce que le polymère est constitué par du dextrane aminé, de préférence de masse moléculaire moyenne en poids d'environ 40 000, les liaisons ioniques étant réalisées par l'intermédiaire de groupes carboxylates de l'acide benzène hexacarboxylique fixé sur le polymère et les liaisons covalentes étant réalisées par l'intermédiaire des autres groupes carboxyliques de l'acide hexacarboxylique non engagés dans les liaisons ioniques sus-définies et des groupes $NH_2$ de l'hémoglobine.

15. Conjugué macromoléculaire selon la revendication 1, caractérisé en ce que le polymère est constitué par du dextrane polycarboxylé, de préférence de masse moléculaire moyenne en poids d'environ 40 000, contenant environ une chaîne de diacide carboxylique par monomère, les liaisons covalentes étant réalisées par l'intermédiaire de groupes carboxyliques non engagés dans la liaison ionique avec l'hémoglobine et des groupes $NH_2$ de l'hémoglobine.

**16.** Procédé de préparation des conjugués macromoléculaires selon la revendication 1, caractérisé en ce que :
- dans une première étape, on fixe les sites Z sur le polymère
  . soit en transformant préalablement le polymère P en polyol, si besoin est, puis on fixe les sites Z par exemple par sulfatation, phosphorylation ou carboxyméthylation ou bien
  . soit en utilisant un composé Z-Y dans lequel Y est une fonction active ou activable, telle que aldéhyde, carboxyle, amine, hydroxyle ou halogène, ou bien
  . soit en effectuant un greffage radiochimique des sites Z sur le polymère P ;
- puis dans une seconde étape, on fait réagir le polymère ci-dessus obtenu avec l'hémoglobine déoxygénée, dans des conditions telles que l'hémoglobine ne subisse pas de dénaturation, en milieu aqueux de pH compris d'environ 5 à environ 9, pour former simultanément les liaisons ioniques et les liaisons covalentes entre le polymère et l'hémoglobine,
- éventuellement lorsque la réaction ci-dessus indiquée conduit à des fonctions imines, celles-ci sont stabilisées en fonctions amines, par exemple par réduction à l'aide de $NaBH_4$ $NaCNBH_3$ le diméthylaminoborane ou HCOOH.

**17.** Procédé selon la revendication 16, caractérisé en ce que à l'issue de la première étape, le polymère comportant les sites Z est activé avant d'être mis en réaction avec l'hémoglobine.

**18.** Procédé selon la revendication 17, caractérisé en ce que l'activation consiste à transformer les fonctions OH en groupes aldéhydes, par exemple par oxydation périodique.

**19.** Procédé selon la revendication 16, caractérisé en ce que l'activation du polymère comportant les sites Z et sa mise en réaction avec l'hémoglobine sont pratiquement simultanées.

**20.** Procédé selon la revendication 19, caractérisé en ce que le polymère comportant les sites Z :
- est activé par exemple à l'aide de bromure de cyanogène ou de carbonyldiimidazole, lorsque ni le polymère P, ni les sites Z comportent de groupes aldéhydes, mais qu'ils contiennent des fonctions hydroxyles.
- ou bien est activé à l'aide de réactifs utilisés en synthèse peptidique, lorsque le polymère P ou les groupes Z comportent des groupes carboxyles,

**21.** Procédé de préparation selon la revendication 20, caractérisé en ce que la réaction entre le polymère et l'hémoglobine pendant un temps inférieur à celui entraînant au plus environ 5 % de méthémoglobine, de préférence pendant au plus 10 h, et à une température permettant une conservation correcte de l'hémoglobine, par exemple entre 3° C et 30° C.

**Revendications pour l'Etat contractant suivant : AT**

**1.** Procédé de préparation de conjugué macromoléculaire hydrosoluble d'hémoglobine, non biodégradable ou peu biodégradable, pendant le temps durant lequel le conjugué macromoléculaire doit assurer des fonctions oxyphoriques dans le plasma, présentant pour l'oxygène une affinité inférieure à celle de l'hémoglobine libre, caractérisé en ce qu'il est constitué :
- d'une part de l'hémoglobine, laquelle peut passer de façon réversible de la forme déoxygénée à la forme oxygénée,
- d'autre part par un polymère P hydrosoluble, non toxique, de préférence non antigénique, hémocompatible, de masse moléculaire d'environ 1 000 à environ 500 000, de préférence d'environ 1 000 à environ 100 000, comportant des groupes polaires, de préférence des groupes hydroxyles, carboxyles ou amines,
- lequel conjugué macromoléculaire comporte des sites Z attachés sur le polymère P et contenant au moins une charge négative portée par des groupes anioniques constitués par des sulfates et/ou phosphates et/ou carboxylates,
- le polymère P étant relié à l'hémoglobine
  . d'une part par l'intermédiaire d'au moins une liaison ionique établie entre l'un au moins des sites Z portés par le polymère et l'hémoglobine, et
  . d'autre part par l'intermédiaire d'au moins une liaison covalente établie, soit entre le polymère et l'hémoglobine, soit entre l'un au moins des sites Z portés par le polymère et l'hémoglobine,
- le nombre de liaisons covalentes entre le polymère et l'hémoglobine étant tel que le conjugué

macromoléculaire a une masse moléculaire moyenne d'environ 70 000 à environ 1 000 000, de préférence d'environ 70 000 à environ 500 000,

- la relation entre la charge négative, le nombre de sites et le nombre de monomères étant la suivante :
  . lorsque chaque site contient un groupe anionique unique constitué d'un sulfate ou d'un phosphate, il y a au moins un site tous les dix monomères du polymère,
  . lorsque l'un au moins des sites contient au moins deux groupes anioniques, constitués de sulfates et/ou de phosphates, il y a au moins un site pour le polymère,
  . lorsque chaque site contient des charges négatives provenant de carboxylates, il faut au moins deux groupes carboxylates sur un même site Z, et au moins un site Z tous les cinq monomères,
  . lorsque l'un au moins des sites contient au moins trois charges négatives provenant de carboxylates, il y a au moins un site pour le polymère, dans lequel :
- dans une première étape, on fixe les sites Z sur le polymère
  . soit en transformant préalablement le polymère P en polyol, si besoin est, puis on fixe les sites Z par exemple par sulfatation, phosphorylation ou carboxyméthylation ou bien
  . soit en utilisant un composé Z-Y dans lequel Y est une fonction active ou activable, telle que aldéhyde, carboxyle, amine, hydroxyle ou halogène, ou bien
  . soit en effectuant un greffage radiochimique des sites Z sur le polymère P ;
- puis dans une seconde étape, on fait réagir le polymère ci-dessus obtenu avec l'hémoglobine déoxygénée, dans des conditions telles que l'hémoglobine ne subisse pas de dénaturation, en milieu aqueux de pH compris d'environ 5 à environ 9, pour former simultanément les liaisons ioniques et les liaisons covalentes entre le polymère et l'hémoglobine,
- éventuellement lorsque la réaction ci-dessus indiquée conduit à des fonctions imines, celles-ci sont stabilisées en fonctions amines, par exemple par réduction à l'aide de $NaBH_4$, $NaCNBH_3$, le diméthylaminoborane ou HCOOH.

2. Procédé de préparation de conjugué macromoléculaire selon la revendication 1, dans lequel les ponts salins entre les groupes $NH_3^+$ et les groupes $COO^-$ internes de l'hémoglobine sont intacts lorsque l'hémoglobine est sous forme déoxygénée.

3. Procédé de préparation de conjugué macromoléculaire selon la revendication 1, dans lequel le polymère P est choisi parmi les polysaccharides, notamment les hydroxyalkylamidons dont le radical alkyle comporte de 2 à 4 atomes de carbone, l'inuline, le dextrane et ses dérivés, notamment le dextrane aminé, l'alcool polyvinylique, la polyvinylpyrrolidone, le polyméthacrylate et ses dérivés, les polypeptides, les polyalkène glycols dans lesquels le groupe alkène comporte de 2 à 5 atomes de carbone, notamment le polyéthylène glycol et le polypropylène glycol.

4. Procédé de préparation de conjugué macromoléculaire selon la revendication 1, dans lequel le polymère P a une masse moléculaire moyenne inférieure ou égale à 70 000, lorsqu'il est constitué par le dextrane et ses dérivés, et une masse moléculaire inférieure ou égale à 10 000 lorsqu'il est choisi parmi les polyalkylène glycols, la polyvinylpyrrolidone ou le polyméthylacrylate.

5. Procédé de préparation de conjugué macromoléculaire selon la revendication 1, dans lequel le site Z est relié au polymère par l'intermédiaire d'une fonction ester, éther, amide ou amine.

6. Procédé de préparation de conjugué macromoléculaire selon la revendication 1, dans lequel le site Z provient de $OSO_3H$, $OPO_3H_2$, $O\text{-}CH(COOH)_2$,

$$-O-CH(COOH)-CH_2-COOH, \quad O-CH_2-\left[\begin{matrix}(CH-CH_2)\\ |\\ COOH\end{matrix}\right]_n-CH_2-COOH,$$

n variant de 1 à environ 4,
ou provient de pyridoxalsulfate, du pyridoxalphosphate, de l'adénosine triphosphate, de la phosphotyrosine, de la phosphosérine, de l'inositolhéxaphosphate et ses dérivés, d'acide polycarboxylique contenant de 2 à 10 atomes de carbone sur la chaîne principale, de l'acide benzènecarboxylique comportant au moins trois fonctions carboxyliques, du 2,3 diphosphoglycérate.

**7.** Procédé de préparation de conjugué macromoléculaire selon la revendication 1, dans lequel l'hémoglobine est liée de façon covalente au polymère par l'intermédiaire de liaisons établies d'une part entre des groupes aldéhydes, des groupes carboxyles, ou des groupes OH et d'autre part des groupes $NH_2$ de l'hémoglobine.

**8.** Procédé de préparation de conjugué macromoléculaire selon la revendication 1, dans lequel les liaisons covalentes entre le polymère et l'hémoglobine sont établies directement entre des groupes aldéhydes, des groupes carboxyles ou des groupes OH portés par le polymère et des groupes $NH_2$ de l'hémoglobine, ou à partir des groupes aldéhydes, carboxyles ou hydroxyles provenant des sites Z fixés sur le polymère et des groupes $NH_2$ de l'hémoglobine.

**9.** Procédé de préparation de conjugué macromoléculaire selon la revendication 1, dans lequel le site Z comporte au moins trois groupes fonctionnels qui sont tels que :

- l'une des fonctions est une fonction permettant de fixer le site Z sur le polymère,
- l'une des fonctions est une fonction sulfate, phosphate ou carboxylate susceptible d'établir une liaison ionique avec une amine du site allostérique de l'hémoglobine,
- l'une des fonctions est une fonction aldéhyde, carboxyle ou OH susceptible de former une liaison covalente avec un groupe $NH_2$ de l'hémoglobine.

**10.** Procédé de préparation de conjugué macromoléculaire selon la revendication 1, dans lequel le polymère est constitué par du dextrane, de préférence de masse moléculaire moyenne en poids d'environ 40 000, contenant de préférence environ 5,4 moles de groupes $OSO_3H$ pour 10 moles de glucopyranose, les liaisons covalentes entre le polymère et l'hémoglobine résultant de la liaison entre des groupes aldéhydes préalablement formés sur le dextrane, de préférence à raison d'environ 18 groupements aldéhydes pour 100 unités glucosidiques et des groupes $NH_2$ de l'hémoglobine.

**11.** Procédé de préparation de conjugué macromoléculaire selon la revendication 1, dans lequel le polymère est constitué par du dextrane, de préférence de masse moléculaire moyenne en poids d'environ 10 000, contenant environ 9,8 moles de groupes $OSO_3H$ pour 10 unités de glucopyranose, les liaisons covalentes entre le polymère et l'hémoglobine résultant de la liaison entre des groupes aldéhydes préalablement formés sur le dextrane, de préférence à raison d'environ 14 groupes aldéhydes pour 100 unités glucosidiques et des groupes $NH_2$ de l'hémoglobine.

**12.** Procédé de préparation de conjugué macromoléculaire selon la revendication 1, dans lequel le polymère est constitué par du dextrane, de préférence de masse moléculaire moyenne en poids d'environ 6 000, contenant environ 1,7 ions sulfates de groupes $OSO_3H$ pour 10 unités de glucopyranose, les liaisons covalentes entre le polymère et l'hémoglobine résultant de la liaison entre des groupes aldéhydes préalablement formés sur le dextrane, de préférence à raison d'environ 18 groupes aldéhydes pour 100 unités.

**13.** Procédé de préparation de conjugué macromoléculaire selon la revendication 1, dans lequel le polymère est constitué par du dextrane, de préférence de masse moléculaire moyenne en poids d'environ 40 000, contenant environ 12 modes de groupes $OPO_3H_2$ pour 10 modes de glucopyranose, les liaisons covalentes entre le polymère et l'hémoglobine résultant de la liaison entre des groupes aldéhydes préalablement formés sur le dextrane, de préférence à raison d'environ 18 groupes aldéhydes pour 100 unités glucosidiques, et des groupes $NH_2$ de l'hémoglobine.

**14.** Procédé de préparation de conjugué macromoléculaire selon la revendication 1, dans lequel le polymère est constitué par du dextrane aminé, de préférence de masse moléculaire moyenne en poids d'environ 40 000, les liaisons ioniques étant réalisées par l'intermédiaire de groupes carboxylates de l'acide benzène hexacarboxylique fixé sur le polymère et les liaisons covalentes étant réalisées par l'intermédiaire des autres groupes carboxyliques de l'acide hexacarboxylique non engagés dans les liaisons ioniques sus-définies et des groupes $NH_2$ de l'hémoglobine.

**15.** Procédé de préparation de conjugué macromoléculaire selon la revendication 1, dans lequel le polymère est constitué par du dextrane polycarboxylé, de préférence de masse moléculaire moyenne en poids d'environ 40 000, contenant environ une chaîne de diacide carboxylique par monomère, les liaisons covalentes étant réalisées par l'intermédiaire de groupes carboxyliques non engagés dans la liaison ionique avec l'hémoglobine et des groupes $NH_2$ de l'hémoglobine.

**16.** Procédé selon l'une des revendications 1 à 15, caractérisé en ce que à l'issue de la première étape, le polymère comportant les sites Z est activé avant d'être mis en réaction avec l'hémoglobine.

**17.** Procédé selon la revendication 16, caractérisé en ce que l'activation consiste à transformer les fonctions OH en groupes aldéhydes, par exemple par oxydation périodique.

**18.** Procédé selon l'une des revendications 1 à 15, caractérisé en ce que l'activation du polymère comportant les sites Z et sa mise en réaction avec l'hémoglobine sont pratiquement simultanées.

**19.** Procédé selon la revendication 18, caractérisé en ce que le polymère comportant les sites Z :
- est activé par exemple à l'aide de bromure de cyanogène ou de carbonyldiimidazole, lorsque ni le polymère P, ni les sites Z comportent de groupes aldéhydes, mais qu'ils contiennent des fonctions hydroxyles,
- ou bien est activé à l'aide de réactifs utilisés en synthèse peptidique, lorsque le polymère P ou les groupes Z comportent des groupes carboxyles.

**20.** Procédé de préparation selon la revendication 19, caractérisé en ce que la réaction entre le polymère et l'hémoglobine pendant un temps inférieur à celui entraînant au plus environ 5% de méthémoglobine, de préférence pendant au plus 10 h, et à une température permettant une conservation correcte de l'hémoglobine, par exemple entre 3$^\circ$ C et 30$^\circ$ C.

**Claims**

**Claims for the following Contracting States : BE, CH, DE, ES, FR, GB, GR, IT, LI, LU, NL, SE**

**1.** Water-soluble macromolecular conjugate of hemoglobin, only slightly or not at all biodegradable during the period of time during which the macromolecular conjugate must exercise its oxygen-carrying functions in the plasma, exhibiting an affinity for oxygen less than that of free hemoglobin, characterized in that it is constituted:
- on the one hand, by hemoglobin, which can undergo transition from the deoxygenated form to the oxygenated form in a reversible manner,
- on the other hand, by a water-soluble polymer P which is nontoxic, preferably non-antigenic, hemocompatible, possessing a molecular weight varying from about 1,000 to about 500,000, and preferably from about 1,000 to about 100,000, and containing polar groups, preferably hydroxyl, carboxyl or amine groups,
- this macromolecular conjugate comprises Z sites bound to the polymer P and containing at least one negative charge carried by anionic groups constituted by sulfate and/or phosphate and/or carboxylate groups,
- the polymer P being bound to the hemoglobin
  . on the one hand through the intermediary of at least one ionic linkage established between at least one of the Z sites carried by the polymer and hemoglobin, and
  . on the other hand, through the intermediary of at least one covalent linkage established either between the polymer and hemoglobin or between at least one of the Z sites carried by the polymer and hemoglobin,
- the number of covalent linkages between the polymer and hemoglobin is such that the macromolecular conjugate has a mean molecular weight varying from about 70,000 to about 1,000,000, and preferably from about 70,000 to about 500,000,
- with the relationship between the negative charge, the number of sites and the number of monomers being the following:
  . when each site contains a unique anionic group constituted by a sulfate or a phosphate residue, there is at least one site for every 10 monomers of the polymer,
  . when at least one of the sites contains at least two anionic groups constituted by sulfate and/or phosphate groups, there is at least one site per polymer,

. when each site contains negative charges which are derived from carboxylate residues, then at least two carboxylate groups must be present at the same Z site and there must be at least one Z site for every five monomers,

. when at least one of the sites contains at least three negative charges which are derived from carboxylate groups, there is at least one site per polymer molecule.

2. Macromolecular conjugate according to Claim 1, characterized in that the salt bridges between the internal $NH_3^-$ groups and the $COO^-$ groups of hemoglobin are intact when hemoglobin is in the deoxygenated form.

3. Macromolecular conjugate according to Claim 1, characterized in that the polymer P is chosen from among the polysaccharides, in particular the hydroxyalkyl starches in which the alkyl radical contains from 2 to 4 carbon atoms, inulin, dextran and its derivatives, in particular aminated dextran, polyvinyl alcohol, polyvinylpyrrolidone, polymethacrylate and its derivatives, polypeptides, polyalkene glycols in which the alkene group contains from 2 to 5 carbon atoms, in particular polyethylene glycol and polypropylene glycol.

4. Macromolecular conjugate according to Claim 1, characterized in that the polymer P has a mean molecular weight lower than, or equal to 70,000, when it is constituted by dextran and its derivatives, and a molecular weight less than, or equal to 10,000 when it is chosen from among the polyalkylene glycols, polyvinylpyrrolidone or polymethacrylate.

5. Macromolecular conjugate according to Claim 1, characterized in that the Z site is bound to the polymer by the intermediary of an ester, ether, amide or amine function.

6. Macromolecular conjugate according to Claim 1, characterized in that the Z site comes from $OSO_3H$, $OPO_3H_2$, $O-CH(COOH)_2$, $-O-CH(COOH)-CH_2-COOH$,

$$O-CH_2-\left[\begin{array}{c}(CH-CH_2)_n\\ |\\ COOH\end{array}\right]-CH_2-COOH,$$

n varying from 1 to about 4, or comes from pyridoxal sulfate, pyridoxal phosphate, adenosine triphosphate, phosphotyrosine, phosphoserine, inositol hexaphosphate and its derivatives, a polycarboxylic acid containing from 2 to 10 carbon atoms in the main chain, a benzene carboxylic acid comprising at least three carboxylic acid functions, 2,3-diphosphoglycerate.

7. Macromolecular conjugate according to Claim 1, characterized in that the hemoglobin is linked covalently to the polymer through the intermediary of linkages established, on the one hand, between aldehyde groups, carboxyl groups or OH groups and, on the other, $NH_2$ groups of hemoglobin.

8. Macromolecular conjugate according to Claim 1, characterized in that the covalent linkages between the polymer and hemoglobin are established directly between aldehyde, carboxyl or OH groups contained in the polymer and $NH_2$ groups of hemoglobin, or between aldehyde, carboxyl or hydroxyl groups coming from the Z sites bound to the polymer and $NH_2$ groups of hemoglobin.

9. Macromolecular conjugate according to Claim 1, characterized in that the Z site contains at least three functional groups which are such that

- one of the functions is a function through which the Z site can be bound to the polymer,
- one of the functions is a sulfate, phosphate or carboxylate function capable of establishing an ionic linkage with an amine at the allosteric site of hemoglobin,
- one of the functions is an aldehyde, carboxyl or OH function capable of forming the covalent link with a $NH_2$ group of hemoglobin.

10. Macromolecular conjugate according to Claim 1, characterized in that the polymer is constituted by dextran, preferably having a mean molecular weight of about 40,000, preferably containing about 5.4

moles of $OSO_3H$ groups per 10 moles of glucopyranose, and the covalent bonds between the polymer and hemoglobin result from the coupling between aldehyde groups previously formed on the dextran, and preferably in the ratio of about 18 aldehyde groups per 100 glucosidic units, and $NH_2$ groups of hemoglobin.

**11.** Macromolecular conjugate according to Claim 1, characterized in that the polymer is constituted by dextran, preferably having a mean molecular weight of about 10,000, containing about 9.8 moles of $OSO_3H$ groups per ten glucopyranose units, and the covalent linkages between the polymer and hemoglobin result from the coupling between aldehyde groups previously formed on the dextran, preferably in a ratio of about 14 aldehyde groups per 100 glucosidic units, and $NH_2$ groups of hemoglobin.

**12.** Macromolecular conjugate according to Claim 1, characterized in that the polymer is constituted by dextran, preferably having a mean molecular weight of about 6,000, containing about 1.7 sulfate $OSO_3H$ groups per 10 units of glucopyranose, and the covalent linkages between the polymer and hemoglobin result from the coupling between aldehyde groups previously formed on the dextran, preferably in a ratio of about 18 aldehyde groups per 100 glucosidic units, and $NH_2$ groups of hemoglobin.

**13.** Macromolecular conjugate according to Claim 1, characterized in that the polymer is constituted by dextran, preferably having a mean molecular weight of about 40,000, containing about 12 moles of $OPO_3H_2$ groups per 10 moles of glucopyranose, and the covalent linkages between the polymer and hemoglobin resulting from the coupling between aldehyde groups previously formed on the dextran, preferably in a ratio of about 18 aldehyde groups per 100 glucosidic units, and $NH_2$ groups of hemoglobin.

**14.** Macromolecular conjugate according to Claim 1, characterized in that the polymer is constituted by aminated dextran, preferably having a mean molecular weight of about 40,000, the ionic linkages being formed through the intermediary of carboxylate residues of the benzene hexacarboxylic acid group attached to the polymer and the covalent linkages being formed through the intermediary of the other carboxylic acid residues of the benzene hexacarboxylic acid not involved in the ionic linkages mentioned above and $NH_2$ groups of hemoglobin.

**15.** Macromolecular conjugate according to Claim 1, characterized in that the polymer is constituted by a polycarboxylated dextran, preferably having a mean molecular weight of about 40,000, containing about one dicarboxylic acid chain per monomer, the covalent linkages being established through the intermediary of carboxylic acid residues not involved in ionic bond formation with hemoglobin and $NH_2$ groups of hemoglobin.

**16.** Procedure for the preparation of the macromolecular conjugates according to Claim 1, characterized in that:
- in the first step, the Z sites are bound to the polymer,
  . either by first transforming the polymer P into a polyol, if necessary, then by attaching the Z sites by sulfatation, phosphorylation or carboxymethylation, for example
  . or by using a compound Z-Y in which Y is a reactive or activatable function such as an aldehyde, carboxyl, amine, hydroxyl or halogen group,
  . or by grafting Z sites on to the polymer P radiochemically;
- then in the second step, the polymer obtained above is allowed to react with deoxygenated hemoglobin in aqueous medium at a pH between about 5 and about 9, conditions under which hemoglobin is not denatured, in order to form simultaneously the ionic and covalent linkages between the polymer and hemoglobin,
- in the case in which the above-mentioned reaction leads to the formation of imine functions, these latter are stabilized by being reduced to amine functions by means of $NaBH_4$, $NaCNBH_3$, dimethylaminoborane or HCOOH.

**17.** Procedure according to Claim 16, characterized in that at the completion of the first step the polymer containing the Z sites is activated before being allowed to react with hemoglobin.

**18.** Procedure according to Claim 17, characterized in that the activation consists of converting the OH

functions into aldehyde groups, for example by periodate oxidation.

**19.** Procedure according to Claim 16, characterized in that the activation of the polymer containing the Z sites and its reaction with hemoglobin are practically simultaneous.

**20.** Procedure according to Claim 19, characterized in that the polymer containing the Z sites:
- is activated for example by means of cyanogen bromide or carbonyldiimidazole, when neither the polymer P nor the Z sites contain aldehyde groups but do contain hydroxyl functions,
- or it is activated using reagents employed in peptide synthesis when the polymer P or the Z groups contain carboxyl residues.

**21.** Preparation procedure according to Claim 20, characterized in that the reaction between the polymer and hemoglobin is carried out for a time shorter than that leading to the formation of more than about 5% of methemoglobin, and preferably not exceeding 10 h, and at a temperature at which hemoglobin is not denatured, for example, between 3° C and 30° C.

**Claims for the following Contracting State : AT**

**1.** Process for preparing a water-soluble macromolecular conjugate of hemoglobin, only slightly or not at all biodegradable during the period of time during which the macromolecular conjugate must exercise its oxygen-carrying functions in the plasma, exhibiting an affinity for oxygen less than that of free hemoglobin, characterized in that it is constituted:
- on the one hand, by hemoglobin, which can undergo transition from the deoxygenated form to the oxygenated form in a reversible manner,
- on the other hand, by a water-soluble polymer P which is nontoxic, preferably non-antigenic, hemocompatible, possessing a molecular weight varying from about 1,000 to about 500,000, and preferably from about 1,000 to about 100,000, and containing polar groups, preferably hydroxyl, carboxyl or amine groups,
- this macromolecular conjugate comprises Z sites bound to the polymer P and containing at least one negative charge carried by anionic groups constituted by sulfate and/or phosphate and/or carboxylate groups,
- the polymer P being bound to the hemoglobin
  . on the one hand through the intermediary of at least one ionic linkage established between at least one of the Z sites carried by the polymer and hemoglobin, and
  . on the other hand, through the intermediary of at least one covalent linkage established either between the polymer and hemoglobin or between at least one of the Z sites carried by the polymer and hemoglobin,
- the number of covalent linkages between the polymer and hemoglobin is such that the macromolecular conjugate has a mean molecular weight varying from about 70,000 to about 1,000,000, and preferably from about 70,000 to about 500,000,
- with the relationship between the negative charge, the number of sites and the number of monomers being the following:
  . when each site contains a unique anionic group constituted by a sulfate or a phosphate residue, there is at least one site for every 10 monomers of the polymer,
  . when at least one of the sites contains at least two anionic groups constituted by sulfate and/or phosphate groups, there is at least one site per polymer,
  . when each site contains negative charges which are derived from carboxylate residues, then at least two carboxylate groups must be present at the same Z site and there must be at least one Z site for every five monomers,
  . when at least one of the sites contains at least three negative charges which are derived from carboxylate groups, there is at least one site per polymer molecule, in which:
- in the first step, the Z sites are bound to the polymer,
  . either by first transforming the polymer P into a polyol, if necessary, then by attaching the Z sites by sulfatation, phosphorylation or carboxymethylation, for example
  . or by using a compound Z-Y in which Y is a reactive or activatable function such as an aldehyde, carboxyl, amine, hydroxyl or halogen group,
  . or by grafting Z sites on to the polymer P radiochemically;
- then in the second step, the polymer obtained above is allowed to react with deoxygenated

hemoglobin in aqueous medium at a pH between about 5 and about 9, conditions under which hemoglobin is not denatured, in order to form simultaneously the ionic and covalent linkages between the polymer and hemoglobin,

- in the case in which the above-mentioned reaction leads to the formation of imine functions, these latter are stabilized by being reduced to amine functions by means of $NaBH_4$, $NaCNBH_3$, dimethylaminoborane or HCOOH.

2. Process for preparing a macromolecular conjugate according to Claim 1, characterized in that the salt bridges between the internal $NH_3^-$ groups and the $COO^-$ groups of hemoglobin are intact when hemoglobin is in the deoxygenated form.

3. Process for preparing a macromolecular conjugate according to Claim 1, characterized in that the polymer P is chosen from among the polysaccharides, in particular the hydroxyalkyl starches in which the alkyl radical contains from 2 to 4 carbon atoms, inulin, dextran and its derivatives, in particular aminated dextran, polyvinyl alcohol, polyvinylpyrrolidone, polymethacrylate and its derivatives, polypeptides, polyalkene glycols in which the alkene group contains from 2 to 5 carbon atoms, in particular polyethylene glycol and polypropylene glycol.

4. Process for preparing a macromolecular conjugate according to Claim 1, characterized in that the polymer P has a mean molecular weight lower than, or equal to 70,000, when it is constituted by dextran and its derivatives, and a molecular weight less than, or equal to 10,000 when it is chosen from among the polyalkylene glycols, polyvinylpyrrolidone or polymethacrylate.

5. Process for preparing a macromolecular conjugate according to Claim 1, characterized in that the Z site is bound to the polymer by the intermediary of an ester, ether, amide or amine function.

6. Process for preparing a macromolecular conjugate according to Claim 1, characterized in that the Z site comes from $OSO_3H$, $OPO_3H_2$, $O\text{-}CH(COOH)_2$, $\text{-}O\text{-}CH(COOH)\text{-}CH_2\text{-}COOH$,

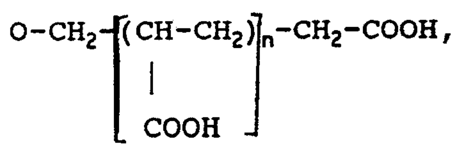

n varying from 1 to about 4, or comes from pyridoxal sulfate, pyridoxal phosphate, adenosine triphosphate, phosphotyrosine, phosphoserine, inositol hexaphosphate and its derivatives, a polycarboxylic acid containing from 2 to 10 carbon atoms in the main chain, a benzene carboxylic acid comprising at least three carboxylic acid functions, 2,3-diphosphoglycerate.

7. Process for preparing a macromolecular conjugate according to Claim 1, characterized in that the hemoglobin is linked covalently to the polymer through the intermediary of linkages established, on the one hand, between aldehyde groups, carboxyl groups or OH groups and, on the other, $NH_2$ groups of hemoglobin.

8. Process for preparing a macromolecular conjugate according to Claim 1, characterized in that the covalent linkages between the polymer and hemoglobin are established directly between aldehyde, carboxyl or OH groups contained in the polymer and $NH_2$ groups of hemoglobin, or between aldehyde, carboxyl or hydroxyl groups coming from the Z sites bound to the polymer and $NH_2$ groups of hemoglobin.

9. Process for preparing a macromolecular conjugate according to Claim 1, characterized in that the Z site contains at least three functional groups which are such that

- one of the functions is a function through which the Z site can be bound to the polymer,
- one of the functions is a sulfate, phosphate or carboxylate function capable of establishing an ionic linkage with an amine at the allosteric site of hemoglobin,
- one of the functions is an aldehyde, carboxyl or OH function capable of forming the covalent link with a $NH_2$ group of hemoglobin.

**10.** Process for preparing a macromolecular conjugate according to Claim 1, characterized in that the polymer is constituted by dextran, preferably having a mean molecular weight of about 40,000, preferably containing about 5.4 moles of $OSO_3H$ groups per 10 moles of glucopyranose, and the covalent bonds between the polymer and hemoglobin result from the coupling between aldehyde groups previously formed on the dextran, and preferably in the ratio of about 18 aldehyde groups per 100 glucosidic units, and $NH_2$ groups of hemoglobin.

**11.** Process for preparing a macromolecular conjugate according to Claim 1, characterized in that the polymer is constituted by dextran, preferably having a mean molecular weight of about 10,000, containing about 9.8 moles of $OSO_3H$ groups per ten glucopyranose units, and the covalent linkages between the polymer and hemoglobin result from the coupling between aldehyde groups previously formed on the dextran, preferably in a ratio of about 14 aldehyde groups per 100 glucosidic units, and $NH_2$ groups of hemoglobin.

**12.** Process for preparing a macromolecular conjugate according to Claim 1, characterized in that the polymer is constituted by dextran, preferably having a mean molecular weight of about 6,000, containing about 1.7 sulfate $OSO_3H$ groups per 10 units of glucopyranose, and the covalent linkages between the polymer and hemoglobin result from the coupling between aldehyde groups previously formed on the dextran, preferably in a ratio of about 18 aldehyde groups per 100 glucosidic units, and $NH_2$ groups of hemoglobin.

**13.** Process for preparing a macromolecular conjugate according to Claim 1, characterized in that the polymer is constituted by dextran, preferably having a mean molecular weight of about 40,000, containing about 12 moles of $OPO_3H_2$ groups per 10 moles of glucopyranose, and the covalent linkages between the polymer and hemoglobin resulting from the coupling between aldehyde groups previously formed on the dextran, preferably in a ratio of about 18 aldehyde groups per 100 glucosidic units, and $NH_2$ groups of hemoglobin.

**14.** Process for preparing a macromolecular conjugate according to Claim 1, characterized in that the polymer is constituted by aminated dextran, preferably having a mean molecular weight of about 40,000, the ionic linkages being formed through the intermediary of carboxylate residues of the benzene hexacarboxylic acid group attached to the polymer and the covalent linkages being formed through the intermediary of the other carboxylic acid residues of the benzene hexacarboxylic acid not involved in the ionic linkages mentioned above and $NH_2$ groups of hemoglobin.

**15.** Process for preparing a macromolecular conjugate according to Claim 1, characterized in that the polymer is constituted by a polycarboxylated dextran, preferably having a mean molecular weight of about 40,000, containing about one dicarboxylic acid chain per monomer, the covalent linkages being established through the intermediary of carboxylic acid residues not involved in ionic bond formation with hemoglobin and $NH_2$ groups of hemoglobin.

**16.** Process according to one of Claims 1 to 15, characterized in that at the completion of the first step the polymer containing the Z sites is activated before being allowed to react with hemoglobin.

**17.** Process according to Claim 16, characterized in that the activation consists of converting the OH functions into aldehyde groups, for example by periodate oxidation.

**18.** Process according to one of Claims 1 to 15, characterized in that the activation of the polymer containing the Z sites and its reaction with hemoglobin are practically simultaneous.

**19.** Process according to Claim 18, characterized in that the polymer containing the Z sites:
- is activated for example by means of cyanogen bromide or carbonyldiimidazole, when neither the polymer P nor the Z sites contain aldehyde groups but do contain hydroxyl functions,
- or it is activated using reagents employed in peptide synthesis when the polymer P or the Z groups contain carboxyl residues.

**20.** Preparation process according to Claim 19, characterized in that the reaction between the polymer and hemoglobin is carried out for a time shorter than that leading to the formation of more than about 5% of

methemoglobin, and preferably not exceeding 10 h, and at a temperature at which hemoglobin is not denatured, for example, between 3° C and 30° C.

**Patentansprüche**

**Patentansprüche für folgende Vertragsstaaten : BE, CH, DE, ES, FR, GB, GR, IT, LI, LU, NL, SE**

1. Wasserlösliches makromolekulares Hämoglobinkonjugat, das nicht oder nur wenig biologisch abbaubar ist während der Zeit, in welcher das makromolekulare Konjugat sauerstoffübertragende Funktionen im Plasma gewährleisten soll, wobei es für den Sauerstoff eine geringere Affinität aufweist als diejenige des freien Hämoglobins, dadurch gekennzeichnet, daß es besteht:
   - einerseits aus Hämoglobin, das in reversibler Art von einer sauerstofffreien in eine sauerstoffhaltige Form wechseln kann,
   - andererseits aus einem wasserlöslichen P-Polymer, das nicht toxisch, vorzugsweise nicht antigen und hämokompatibel ist, ein Molekulargewicht von etwa 1 000 bis etwa 500 000, vorzugsweise von etwa 1 000 bis etwa 100 000, aufweist und polare Gruppen, vorzugsweise Hydroxyl-, Carboxyl- oder Amingruppen enthält,
   - wobei das makromolekulare Konjugat an dem P-Polymer befestigte Z-Stellen aufweist und diese wenigstens eine negative Ladung in Form von anionischen Gruppen wie Sulfaten und/oder Phosphaten und/oder Carboxylaten enthalten,
   - wobei das P-Polymer mit dem Hämoglobin verbunden ist,
     * einerseits durch die Zwischenschaltung wenigstens einer ionischen Bindung, die zwischen wenigstens einer der von dem Polymer und dem Hämoglobin getragenen Z-Stellen gebildet ist, und
     * andererseits durch die Zwischenschaltung wenigstens einer kovalenten Bindung, die entweder zwischen dem Polymer und dem Hämoglobin oder zwischen wenigstens einer der von dem Polymer und dem Hämoglobin getragenen Z-Stellen gebildet ist,
   - wobei die Anzahl der kovalenten Bindungen zwischen dem Polymer und dem Hämoglobin so groß ist, daß das makromolekulare Konjugat eine mittlere Molekularmasse von etwa 70 000 bis etwa 1 000 000, vorzugsweise von etwa 70 000 bis etwa 500 000, aufweist,
   - wobei das Verhältnis zwischen der negativen Ladung, der Anzahl der Bindungsstellen und der Anzahl der Monomere folgendermaßen ist:
     * wenn jede Stelle eine anionische Gruppe enthält, die nur aus einem Sulfat oder einem Phosphat besteht, so gibt es zumindest eine Stelle je zehn Monomere des Polymers,
     * wenn wenigstens eine der Stellen wenigstens zwei anionische Gruppen enthält, die aus Sulfaten und/oder Phosphaten gebildet sind, so gibt es wenigstens eine Stelle für das Polymer,
     * wenn jede Stelle aus Carboxylaten stammende negative Ladungen enthält, sind wenigstens zwei Carboxylatgruppen auf derselben Z-Stelle und wenigstens eine Z-Stelle je fünf Monomere notwendig,
     * wenn wenigstens eine der Stellen mindestens drei aus Carboxylaten stammende negative Ladungen aufweist, so gibt es wenigstens eine Stelle für das Polymer.

2. Makromolekulares Konjugat nach Anspruch 1, dadurch gekennzeichnet, daß die Salzbrücken zwischen den internen $NH_3^+$ -Gruppen und den $COO^-$ -Gruppen des Hämoglobins intakt sind, wenn das Hämoglobin in sauerstofffreier Form vorliegt.

3. Makromolekulares Konjugat nach Anspruch 1, dadurch gekennzeichnet, daß das P-Polymer ausgewählt ist aus den Polysacchariden, insbesondere aus den Hydroxyalkylstärken, deren Alkylgruppe 2 bis 4 Kohlenstoffatome enthält, dem Inulin, dem Dextran und seinen Derivaten, insbesondere dem Aminodextran, dem Polyvinylalkohol, dem Polyvinylpyrrolidon, dem Polymethacrylat und seinen Derivaten, den Polypeptiden, den Polyalkenglykolen, deren Alkengruppe 2 bis 5 Kohlenstoffatome enthält, insbesondere dem Polyethylenglykol und dem Polypropylenglykol.

4. Makromolekulares Konjugat nach Anspruch 1, dadurch gekennzeichnet, daß das P-Polymer eine mittlere Molekularmasse von unter oder gleich 70 000 aufweist, wenn es aus dem Dextran und seinen Derivaten gebildet ist, und eine Molekularmasse unter oder gleich 10 000, wenn es aus den Polyalkylenglykolen, dem Polyvinylpyrrolidon oder dem Polymethylacrylat gewählt ist.

5. Makromolekulares Konjugat nach Anspruch 1, dadurch gekennzeichnet, daß die Z-Stelle an das

Polymer durch eine intermediäre Ester-, Ether-, Amid- oder Aminfunktion gebunden ist.

6. Makromolekulares Konjugat nach Anspruch 1, dadurch gekennzeichnet, daß die Z-Stelle aus $OSO_3H$, $OPO_3H_2$, $O-CH(COOH)_2$, $-O-CH(COOH)-CH_2-COOH$,

$$O-CH_2-\left[\begin{array}{c}(CH-CH_2)\\|\\COOH\end{array}\right]_n-CH_2-COOH$$

besteht, wobei n von 1 bis etwa 4 variieren kann,
oder aus Pyridoxalsulfat, Pyridoxalphosphat, Adenosintriphosphat, Tyrosinphosphat, Phosphoserin, aus Inositolhexaphosphat und seinen Derivaten, aus Polycarboxylsäure mit 2 bis 10 Kohlenstoffatomen in der Hauptkette, aus Benzolcarbonsäure, welche mindestens drei Carboxylfunktionen trägt, oder aus 2,3-Diphosphoglyzerat besteht.

7. Makromolekulares Konjugat nach Anspruch 1, bei dem das Hämoglobin kovalent an das Polymer gebunden ist durch intermediäre Bindungen, die einerseits durch Aldehydgruppen, Carboxylgruppen oder OH-Gruppen und andererseits von $NH_2$-Gruppen des Hämoglobins gebildet werden.

8. Makromolekulares Konjugat nach Anspruch 1, dadurch gekennzeichnet, daß die kovalenten Bindungen zwischen dem Polymer und dem Hämoglobin direkt zwischen den Aldehydgruppen, den Carboxylgruppen oder den von dem Polymer getragenen OH-Gruppen und den $NH_2$-Gruppen des Hämoglobins gebildet werden, oder ausgehend von Aldehyd-, Carboxyl- oder Hydroxylgruppen, die von auf dem Polymer und den $NH_2$-Gruppen des Hämoglobins befestigten Z-Stellen stammen.

9. Makromolekulares Konjugat nach Anspruch 1, dadurch gekennzeichnet, daß die Z-Stelle mindestens drei funktionelle Gruppen wie folgt enthält:
   - eine der Funktionen ist eine Funktion, die die Befestigung der Z-Stelle auf dem Polymer erlaubt,
   - eine der Funktionen ist eine Sulfat-, Phosphat- oder Carboxylatfunktion, die geeignet ist, eine ionische Bindung mit einem Amin der allosterischen Stelle des Hämoglobins zu bilden,
   - eine der Funktionen ist eine Aldehyd-, Carboxyl- oder OH-Funktion, die geeignet ist, eine kovalente Bindung mit einer $NH_2$-Gruppe des Hämoglobins zu bilden.

10. Makromolekulares Konjugat nach Anspruch 1, dadurch gekennzeichnet, daß das Polymer aus Dextran gebildet ist, vorzugsweise mit einer mittleren Molekulargewichtsmasse von etwa 40 000, das vorzugsweise etwa 5,4 Mole von $OSO_3H$-Gruppen auf 10 Mole Glucopyranose enthält, wobei die kovalenten Bindungen zwischen dem Polymer und dem Hämoglobin aus der Bindung zwischen Aldehydgruppen, die zuvor auf dem Dextran gebildet wurden, vorzugsweise etwa 18 Aldehydgruppierungen pro 100 Glucose-Einheiten, und $NH_2$-Gruppen des Hämoglobins resultieren.

11. Makromolekulares Konjugat nach Anspruch 1, dadurch gekennzeichnet, daß das Polymer aus Dextran gebildet ist, vorzugsweise mit einer mittleren Molekulargewichtsmasse von etwa 10 000, das vorzugsweise etwa 9,8 Mole von $OSO_3H$-Gruppen auf 10 Mole Glucopyranose enthält, wobei die kovalenten Bindungen zwischen dem Polymer und dem Hämoglobin aus der Bindung zwischen Aldehydgruppen, die zuvor auf dem Dextran gebildet wurden, vorzugsweise etwa 14 Aldehydgruppierungen pro 100 Glucose-Einheiten, und $NH_2$-Gruppen des Hämoglobins resultieren.

12. Makromolekulares Konjugat nach Anspruch 1, dadurch gekennzeichnet, daß das Polymer aus Dextran gebildet ist, vorzugsweise mit einer mittleren Molekulargewichtsmasse von etwa 6 000, das etwa 1,7 Sulfationen als $OSO_3H$-Gruppen auf 10 Einheiten Glucopyranose enthält, wobei die kovalenten Bindungen zwischen dem Polymer und dem Hämoglobin aus der Bindung zwischen Aldehydgruppen, die zuvor auf dem Dextran gebildet wurden, vorzugsweise etwa 18 Aldehydgruppierungen pro 100 Glucose-Einheiten, und $NH_2$-Gruppen des Hämoglobins resultieren.

13. Makromolekulares Konjugat nach Anspruch 1, dadurch gekennzeichnet, daß das Polymer aus Dextran gebildet ist, vorzugsweise mit einer mittleren Molekulargewichtsmasse von etwa 40 000, das etwa 12 Mole $OPO_3H_2$-Gruppen auf 10 Mole Glucopyranose enthält, wobei die kovalenten Bindungen zwischen

dem Polymer und dem Hämoglobin aus der Bindung zwischen Aldehydgruppen, die zuvor auf dem Dextran gebildet wurden, vorzugsweise etwa 18 Aldehydgruppen pro 100 Glucose-Einheiten, und $NH_2$-Gruppen des Hämoglobins resultieren.

**14.** Makromolekulares Konjugat nach Anspruch 1, dadurch gekennzeichnet, daß das Polymer aus Aminodextran gebildet ist, vorzugsweise mit einer mittleren Molekulargewichtsmasse von etwa 40 000, wobei die ionischen Bindungen durch Carboxylatgruppen der an das Polymer gebundenen Benzolhexacarbonsäure gebildet werden und die kovalenten Bindungen durch andere Carboxylgruppen der Benzolhexacarbonsäure, die nicht an den oben definierten ionischen Bindungen beteiligt sind, und von $NH_2$-Gruppen des Hämoglobins gebildet werden.

**15.** Makromolekulares Konjugat nach Anspruch 1, dadurch gekennzeichnet, daß das Polymer aus Polycarboxyldextran gebildet ist, vorzugsweise mit einer mittleren Molekulargewichtsmasse von etwa 40 000, das ungefähr eine Dicarbonsäurekette pro Monomer enthält, wobei die kovalenten Bindungen durch Carboxylgruppen, die nicht an der ionischen Bindung mit dem Hämoglobin beteiligt sind, und von $NH_2$-Gruppen des Hämoglobins gebildet werden.

**16.** Verfahren zur Herstellung von makromolekularen Konjugaten nach Anspruch 1, dadurch gekennzeichnet, daß:
- man in einem ersten Schritt die Z-Stellen auf dem Polymer befestigt
  * sei es durch Transformieren vorzugsweise des P-Polymers in Polyol, falls notwendig, und Befestigen der Z-Stellen zum Beispiel durch Sulfatierung, Phosphorylierung oder Carboxymethylierung, oder
  * sei es durch Verwendung einer Z-Y-Verbindung, in der Y eine aktive oder aktivierbare Gruppe wie ein Aldehyd, Carboxyl, Amin, Hydroxyl oder Halogen darstellt, oder
  * sei es durch eine radiochemische Veredelung der Z-Stellen auf dem P-Polymer;
- man dann in einem zweiten Schritt das nach obigem Schritt erhaltene Polymer mit einem sauerstofffreien Hämoglobin unter solchen Bedingungen, daß das Hämoglobin keine Denaturierung erfährt, in wässriger Umgebung mit einem pH-Wert von etwa 5 bis etwa 9 reagieren läßt, so daß gleichzeitig die ionischen Bindungen und die kovalenten Bindungen zwischen dem Polymer und dem Hämoglobin gebildet werden,
- schließlich, wenn die oben genannte Reaktion zu Imingruppen führt, diese als Aminogruppen stabilisiert werden, zum Beispiel durch Reduktion mit Hilfe von $NaBH_4$, $NaCNBH_3$, Dimethylaminoboran oder HCOOH.

**17.** Verfahren nach Anspruch 16, dadurch gekennzeichnet, daß als Ergebnis des ersten Schrittes das die Z-Stellen enthaltende Polymer aktiviert wird, bevor es mit dem Hämoglobin zur Reaktion gebracht wird.

**18.** Verfahren nach Anspruch 17, dadurch gekennzeichnet, daß die Aktivierung aus dem Transformieren der OH-Gruppen in Aldehydgruppen, beispielsweise durch Periodat-Oxidation, besteht.

**19.** Verfahren nach Anspruch 16, dadurch gekennzeichnet, daß die Aktivierung des die Z-Stellen enthaltenden Polymers und dessen Reaktion mit dem Hämoglobin praktisch gleichzeitig durchgeführt werden.

**20.** Verfahren nach Anspruch 19, dadurch gekennzeichnet, daß das die Z-Stellen enthaltende Polymer:
- aktiviert wird zum Beispiel mit Hilfe von Bromcyan oder Carbonyldiimidazolbromid, wenn weder das P-Polymer noch die Z-Stellen Aldehydgruppen enthalten, aber Hydroxylfunktionen aufweisen,
- oder auch aktiviert wird mit Hilfe von bei der Peptidsynthese verwendeter Reagenzien, wenn das P-Polymer oder die Z-Gruppen Carboxylgruppen enthalten.

**21.** Verfahren nach Anspruch 20, dadurch gekennzeichnet, daß die Reaktion zwischen dem Polymer und dem Hämoglobin während einer Zeit, die geringer ist als jene zum Bilden von mehr als etwa 5% Methämoglobin, vorzugsweise während mehr als 10 Stunden, und bei einer Temperatur, die die korrekte Konservierung des Hämoglobins gestattet, beispielsweise zwischen 3° C und 30° C, stattfindet.

**Patentansprüche für folgenden Vertragsstaat : AT**

1. Verfahren zur Herstellung eines wasserlöslichen makromolekularen Hämoglobinkonjugats, das nicht oder nur wenig biologisch abbaubar ist während der Zeit, in welcher das makromolekulare Konjugat sauerstoffübertragende Funktionen im Plasma gewährleisten soll, wobei es für den Sauerstoff eine geringere Affinität aufweist als diejenige des freien Hämoglobins, dadurch gekennzeichnet, daß es besteht:
   - einerseits aus Hämoglobin, das in reversibler Art von einer sauerstofffreien in eine sauerstoffhaltige Form wechseln kann,
   - andererseits aus einem wasserlöslichen P-Polymer, das nicht toxisch, vorzugsweise nicht antigen und hämokompatibel ist, ein Molekulargewicht von etwa 1 000 bis etwa 500 000, vorzugsweise von etwa 1 000 bis etwa 100 000, aufweist und polare Gruppen, vorzugsweise Hydroxyl-, Carboxyl- oder Amingruppen enthält,
   - wobei das makromolekulare Konjugat an dem P-Polymer befestigte Z-Stellen aufweist und diese wenigstens eine negative Ladung in Form von anionischen Gruppen wie Sulfaten und/oder Phosphaten und/oder Carboxylaten enthalten,
   - wobei das P-Polymer mit dem Hämoglobin verbunden ist,
     * einerseits durch die Zwischenschaltung wenigstens einer ionischen Bindung, die zwischen wenigstens einer der von dem Polymer und dem Hämoglobin getragenen Z-Stellen gebildet ist, und
     * andererseits durch die Zwischenschaltung wenigstens einer kovalenten Bindung, die entweder zwischen dem Polymer und dem Hämoglobin oder zwischen wenigstens einer der von dem Polymer und dem Hämoglobin getragenen Z-Stellen gebildet ist,
   - wobei die Anzahl der kovalenten Bindungen zwischen dem Polymer und dem Hämoglobin so groß ist, daß das makromolekulare Konjugat eine mittlere Molekularmasse von etwa 70 000 bis etwa 1 000 000, vorzugsweise von etwa 70 000 bis etwa 500 000, aufweist,
   - wobei das Verhältnis zwischen der negativen Ladung, der Anzahl der Bindungsstellen und der Anzahl der Monomere folgendermaßen ist:
     * wenn jede Stelle eine anionische Gruppe enthält, die nur aus einem Sulfat oder einem Phosphat besteht, so gibt es zumindest eine Stelle je zehn Monomere des Polymers,
     * wenn wenigstens eine der Stellen wenigstens zwei anionische Gruppen enthält, die aus Sulfaten und/oder Phosphaten gebildet sind, so gibt es wenigstens eine Stelle für das Polymer,
     * wenn jede Stelle aus Carboxylaten stammende negative Ladungen enthält, sind wenigstens zwei Carboxylatgruppen auf derselben Z-Stelle und wenigstens eine Z-Stelle je fünf Monomere notwendig,
     * wenn wenigstens eine der Stellen mindestens drei aus Carboxylaten stammende negative Ladungen aufweist, so gibt es wenigstens eine Stelle für das Polymer,

   worin
   - man in einem ersten Schritt die Z-Stellen auf dem Polymer befestigt
     * sei es durch Transformieren vorzugsweise des P-Polymers in Polyol, falls notwendig, und Befestigen der Z-Stellen zum Beispiel durch Sulfatierung, Phosphorylierung oder Carboxymethylierung, oder
     * sei es durch Verwendung einer Z-Y-Verbindung, in der Y eine aktive oder aktivierbare Gruppe wie ein Aldehyd, Carboxyl, Amin, Hydroxyl oder Halogen darstellt, oder
     * sei es durch eine radiochemische Veredelung der Z-Stellen auf dem P-Polymer;
   - man dann in einem zweiten Schritt das nach obigem Schritt erhaltene Polymer mit einem sauerstofffreien Hämoglobin unter solchen Bedingungen, daß das Hämoglobin keine Denaturierung erfährt, in wässriger Umgebung mit einem pH-Wert von etwa 5 bis etwa 9 reagieren läßt, so daß gleichzeitig die ionischen Bindungen und die kovalenten Bindungen zwischen dem Polymer und dem Hämoglobin gebildet werden,
   - schließlich, wenn die oben genannte Reaktion zu Imingruppen führt, diese als Aminogruppen stabilisiert werden, zum Beispiel durch Reduktion mit Hilfe von $NaBH_4$, $NaCNBH_3$, Dimethylaminoboran oder HCOOH.

2. Verfahren zur Herstellung eines makromolekularen Konjugats nach Anspruch 1, in welchem die Salzbrücken zwischen den internen $NH_3^+$ -Gruppen und den $COO^-$ -Gruppen des Hämoglobins intakt sind, wenn das Hämoglobin in sauerstofffreier Form vorliegt.

3. Verfahren zur Herstellung eines makromolekularen Konjugats nach Anspruch 1, in welchem das P-Polymer ausgewählt ist aus den Polysacchariden, insbesondere aus den Hydroxyalkylstärken, deren

Alkylgruppe 2 bis 4 Kohlenstoffatome enthält, dem Inulin, dem Dextran und seinen Derivaten, insbesondere dem Aminodextran, dem Polyvinylalkohol, dem Polyvinylpyrrolidon, dem Polymethacrylat und seinen Derivaten, den Polypeptiden, den Polyalkenglykolen, deren Alkengruppe 2 bis 5 Kohlenstoffatome enthält, insbesondere dem Polyethylenglykol und dem Polypropylenglykol.

**4.** Verfahren zur Herstellung eines makromolekularen Konjugats nach Anspruch 1, bei dem das P-Polymer eine mittlere Molekularmasse von unter oder gleich 70 000 aufweist, wenn es aus dem Dextran und seinen Derivaten gebildet ist, und eine Molekularmasse unter oder gleich 10 000, wenn es aus den Polyalkylenglykolen, dem Polyvinylpyrrolidon oder dem Polymethacrylat gewählt ist.

**5.** Verfahren zur Herstellung eines makromolekularen Konjugats nach Anspruch 1, bei dem die Z-Stelle an das Polymer durch eine intermediäre Ester-, Ether-, Amid- oder Aminfunktion gebunden ist.

**6.** Verfahren zur Herstellung eines makromolekularen Konjugats nach Anspruch 1, bei dem die Z-Stelle aus $OSO_3H$, $OPO_3H_2$, $O-CH(COOH)_2$, $-O-CH(COOH)-CH_2-COOH$,

$$O-CH_2-\left[\begin{array}{c}(CH-CH_2)\\ |\\ COOH\end{array}\right]_n-CH_2-COOH$$

besteht, wobei n von 1 bis etwa 4 variieren kann,
oder aus Pyridoxalsulfat, Pyridoxalphosphat, Adenosintriphosphat, Tyrosinphosphat, Phosphoserin, aus Inositolhexaphosphat und seinen Derivaten, aus Polycarboxylsäure mit 2 bis 10 Kohlenstoffatomen in der Hauptkette, aus Benzolcarbonsäure, welche mindestens drei Carboxylfunktionen trägt, oder aus 2,3-Diphosphoglyzerat besteht.

**7.** Verfahren zur Herstellung eines makromolekularen Konjugats nach Anspruch 1, bei dem das Hämoglobin kovalent an das Polymer gebunden ist durch intermediäre Bindungen, die einerseits durch Aldehydgruppen, Carboxylgruppen oder OH-Gruppen und andererseits von $NH_2$-Gruppen des Hämoglobins gebildet werden.

**8.** Verfahren zur Herstellung eines makromolekularen Konjugats nach Anspruch 1, bei dem die kovalenten Bindungen zwischen dem Polymer und dem Hämoglobin direkt zwischen den Aldehydgruppen, den Carboxylgruppen oder den von dem Polymer getragenen OH-Gruppen und den $NH_2$-Gruppen des Hämoglobins gebildet werden, oder ausgehend von Aldehyd-, Carboxyl- oder Hydroxylgruppen, die von auf dem Polymer und den $NH_2$-Gruppen des Hämoglobins befestigten Z-Stellen stammen.

**9.** Verfahren zur Herstellung eines makromolekularen Konjugats nach Anspruch 1, bei dem die Z-Stelle mindestens drei funktionelle Gruppen wie folgt enthält:
- eine der Funktionen ist eine Funktion, die die Befestigung der Z-Stelle auf dem Polymer erlaubt,
- eine der Funktionen ist eine Sulfat-, Phosphat- oder Carboxylatfunktion, die geeignet ist, eine ionische Bindung mit einem Amin der allosterischen Stelle des Hämoglobins zu bilden,
- eine der Funktionen ist eine Aldehyd-, Carboxyl- oder OH-Funktion, die geeignet ist, eine kovalente Bindung mit einer $NH_2$-Gruppe des Hämoglobins zu bilden.

**10.** Verfahren zur Herstellung eines makromolekularen Konjugats nach Anspruch 1, bei dem das Polymer aus Dextran gebildet ist, vorzugsweise mit einer mittleren Molekulargewichtsmasse von etwa 40 000, das vorzugsweise etwa 5,4 Mole von $OSO_3H$-Gruppen auf 10 Mole Glucopyranose enthält, wobei die kovalenten Bindungen zwischen dem Polymer und dem Hämoglobin aus der Bindung zwischen Aldehydgruppen, die zuvor auf dem Dextran gebildet wurden, vorzugsweise etwa 18 Aldehydgruppierungen pro 100 Glucose-Einheiten, und $NH_2$-Gruppen des Hämoglobins resultieren.

**11.** Verfahren zur Herstellung eines makromolekularen Konjugats nach Anspruch 1, bei dem das Polymer aus Dextran gebildet ist, vorzugsweise mit einer mittleren Molekulargewichtsmasse von etwa 10 000, das vorzugsweise etwa 9,8 Mole von $OSO_3H$-Gruppen auf 10 Mole Glucopyranose enthält, wobei die kovalenten Bindungen zwischen dem Polymer und dem Hämoglobin aus der Bindung zwischen Aldehydgruppen, die zuvor auf dem Dextran gebildet wurden, vorzugsweise etwa 14 Aldehydgruppierungen pro 100 Glucose-Einheiten, und $NH_2$-Gruppen des Hämoglobins resultieren.

**12.** Verfahren zur Herstellung eines makromolekularen Konjugats nach Anspruch 1, bei dem das Polymer aus Dextran gebildet ist, vorzugsweise mit einer mittleren Molekulargewichtsmasse von etwa 6 000, das etwa 1,7 Sulfationen als $OSO_3H$-Gruppen auf 10 Einheiten Glucopyranose enthält, wobei die kovalenten Bindungen zwischen dem Polymer und dem Hämoglobin aus der Bindung zwischen Aldehydgruppen, die zuvor auf dem Dextran gebildet wurden, vorzugsweise etwa 18 Aldehydgruppierungen pro 100 Glucose-Einheiten, und $NH_2$-Gruppen des Hämoglobins resultieren.

**13.** Verfahren zur Herstellung eines makromolekularen Konjugats nach Anspruch 1, bei dem das Polymer aus Dextran gebildet ist, vorzugsweise mit einer mittleren Molekulargewichtsmasse von etwa 40 000, das etwa 12 Mole $OPO_3H_2$-Gruppen auf 10 Mole Glucopyranose enthält, wobei die kovalenten Bindungen zwischen dem Polymer und dem Hämoglobin aus der Bindung zwischen Aldehydgruppen, die zuvor auf dem Dextran gebildet wurden, vorzugsweise etwa 18 Aldehydgruppen pro 100 Glucose-Einheiten, und $NH_2$-Gruppen des Hämoglobins resultieren.

**14.** Verfahren zur Herstellung eines makromolekularen Konjugats nach Anspruch 1, bei dem das Polymer aus Aminodextran gebildet ist, vorzugsweise mit einer mittleren Molekulargewichtsmasse von etwa 40 000, wobei die ionischen Bindungen durch Carboxylatgruppen der an das Polymer gebundenen Benzolhexacarbonsäure gebildet werden und die kovalenten Bindungen durch andere Carboxylgruppen der Benzolhexacarbonsäure, die nicht an den oben definierten ionischen Bindungen beteiligt sind, und von $NH_2$-Gruppen des Hämoglobins gebildet werden.

**15.** Verfahren zur Herstellung eines makromolekularen Konjugats nach Anspruch 1, bei dem das Polymer aus Polycarboxyldextran gebildet ist, vorzugsweise mit einer mittleren Molekulargewichtsmasse von etwa 40 000, das ungefähr eine Dicarbonsäurekette pro Monomer enthält, wobei die kovalenten Bindungen durch Carboxylgruppen, die nicht an der ionischen Bindung mit dem Hämoglobin beteiligt sind, und von $NH_2$-Gruppen des Hämoglobins gebildet werden.

**16.** Verfahren nach einem der Ansprüche 1 bis 15, dadurch gekennzeichnet, daß als Ergebnis des ersten Schrittes das die Z-Stellen enthaltende Polymer aktiviert wird, bevor es mit dem Hämoglobin zur Reaktion gebracht wird.

**17.** Verfahren nach Anspruch 16, dadurch gekennzeichnet, daß die Aktivierung aus dem Transformieren der OH-Gruppen in Aldehydgruppen, beispielsweise durch Periodat-Oxidation, besteht.

**18.** Verfahren nach einem der Ansprüche 1 bis 15, dadurch gekennzeichnet, daß die Aktivierung des die Z-Stellen enthaltenden Polymers und dessen Reaktion mit dem Hämoglobin praktisch gleichzeitig durchgeführt werden.

**19.** Verfahren nach Anspruch 18, dadurch gekennzeichnet, daß das die Z-Stellen enthaltende Polymer:
- aktiviert wird zum Beispiel mit Hilfe von Bromcyan oder Carbonyldiimidazolbromid, wenn weder das P-Polymer noch die Z-Stellen Aldehydgruppen enthalten, aber Hydroxylfunktionen aufweisen,
- oder auch aktiviert wird mit Hilfe von bei der Peptidsynthese verwendeter Reagenzien, wenn das P-Polymer oder die Z-Gruppen Carboxylgruppen enthalten.

**20.** Verfahren nach Anspruch 19, dadurch gekennzeichnet, daß die Reaktion zwischen dem Polymer und dem Hämoglobin während einer Zeit, die geringer ist als jene zum Bilden von mehr als etwa 5% Methämoglobin, vorzugsweise während mehr als 10 Stunden, und bei einer Temperatur, die die korrekte Konservierung des Hämoglobins gestattet, beispielsweise zwischen 3° C und 30° C, stattfindet.

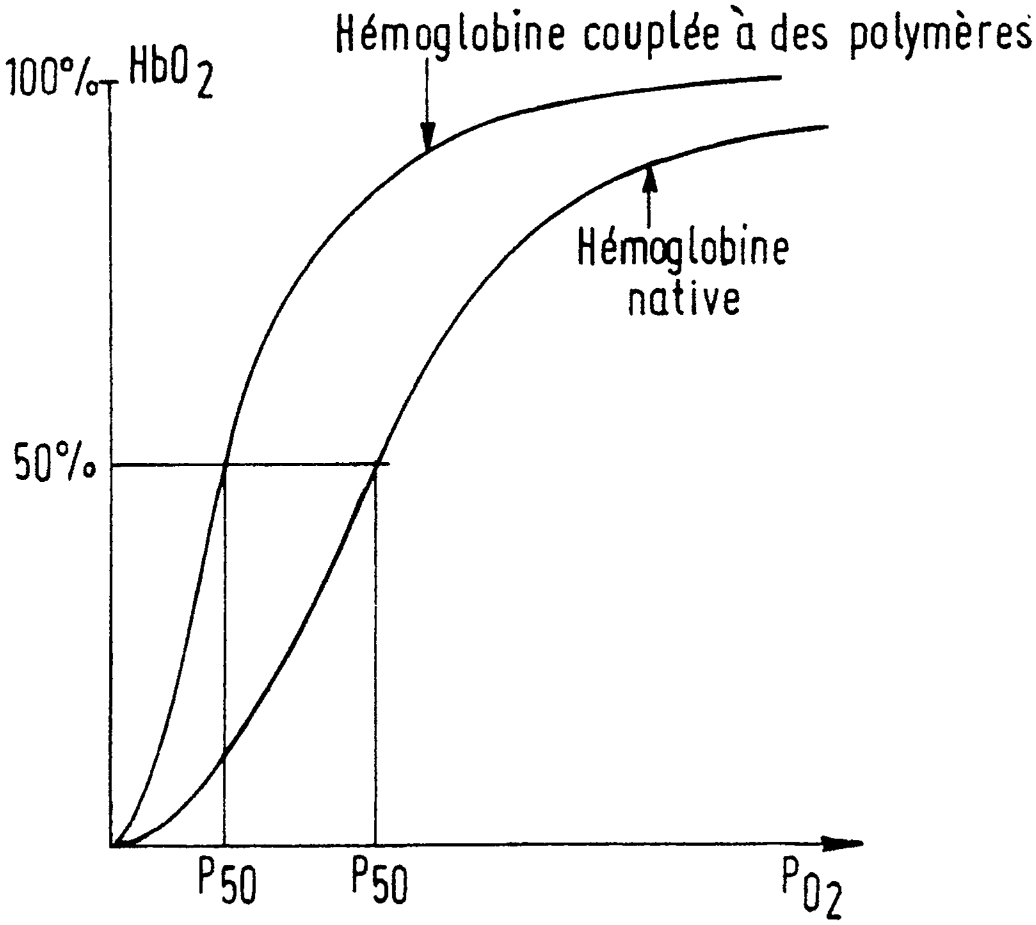
Hémoglobine couplée à des polymères
100% HbO2
Hémoglobine
native
50%
P50
P50
PO2

FIG.1

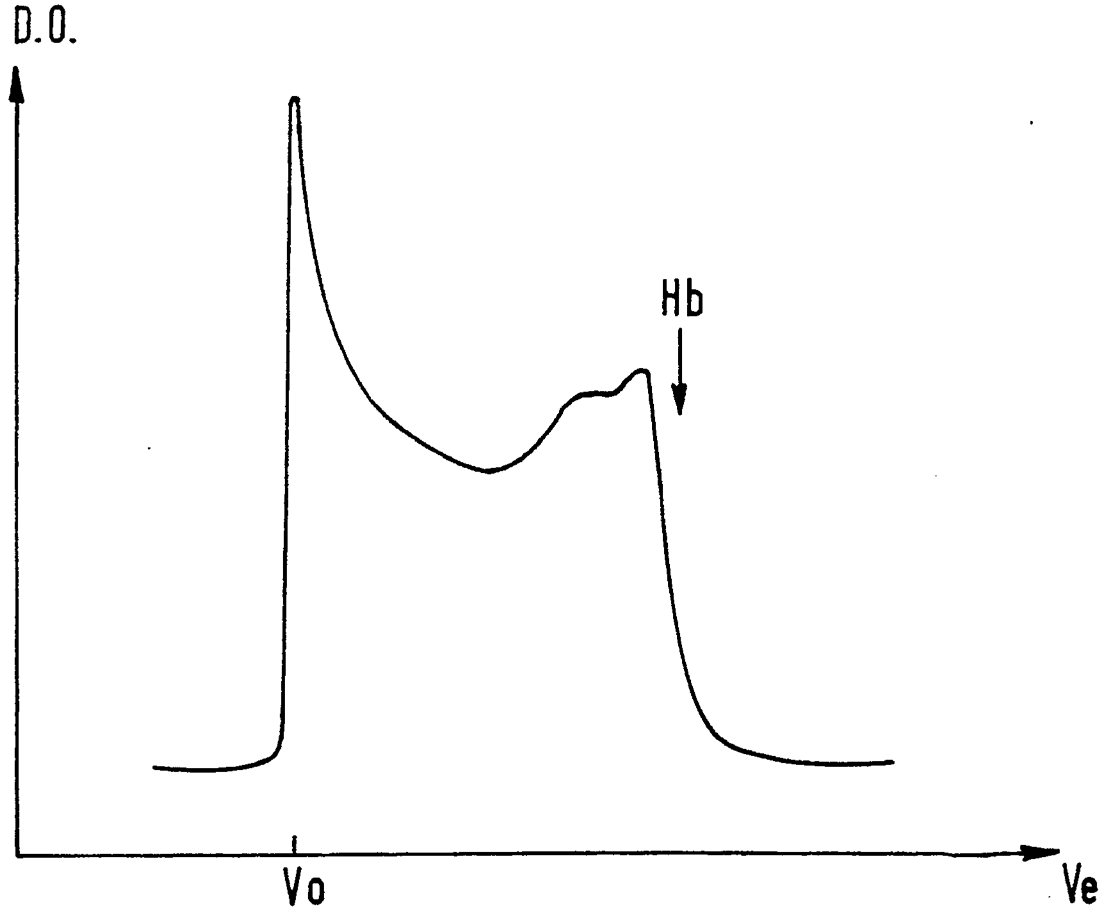


FIG.2

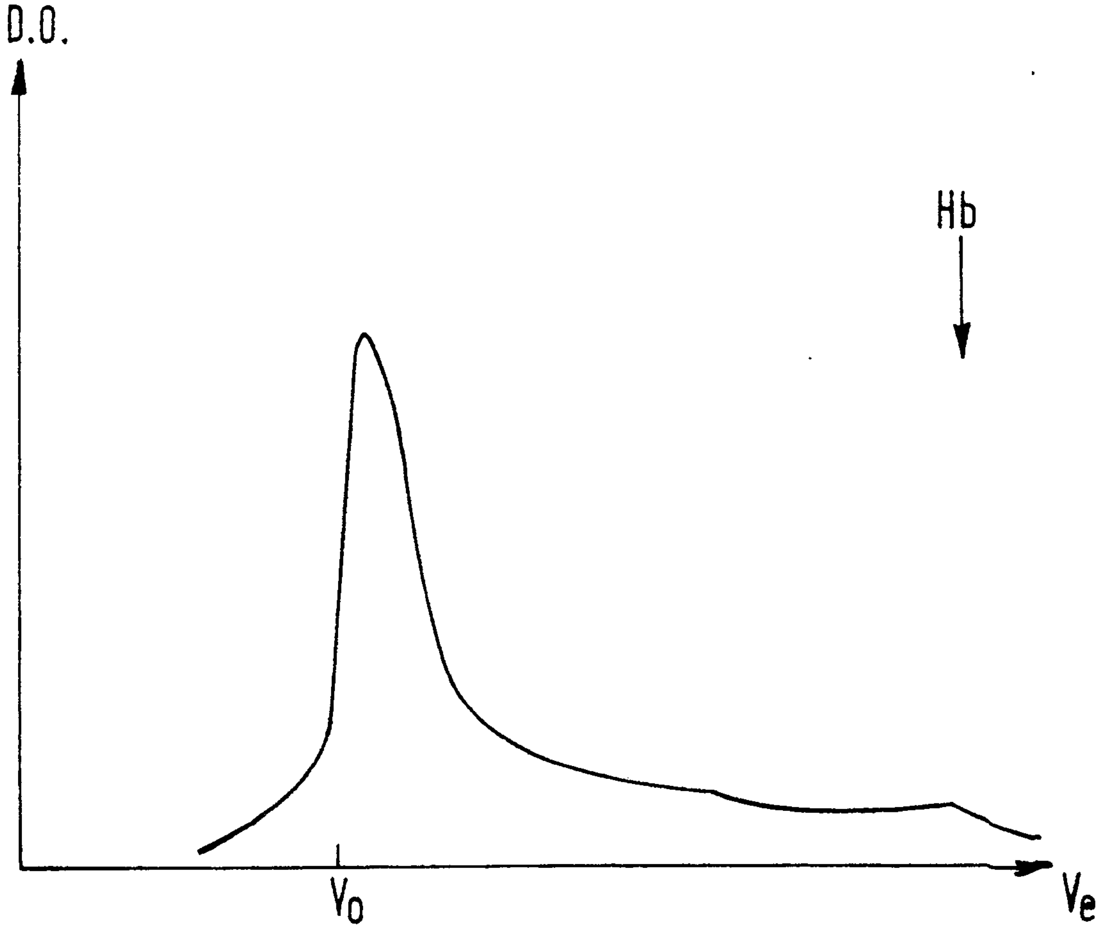


FIG.3

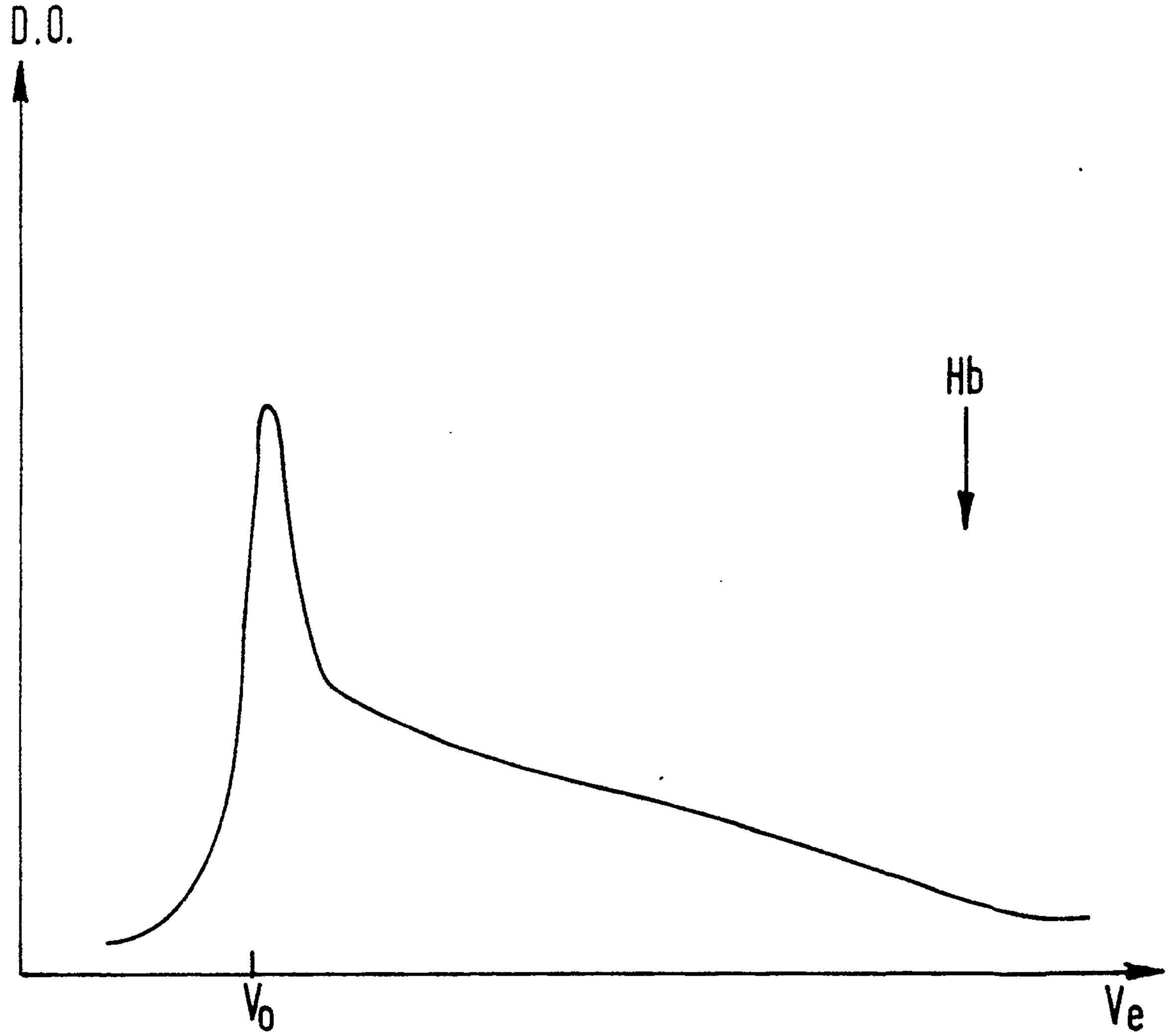


FIG. 4

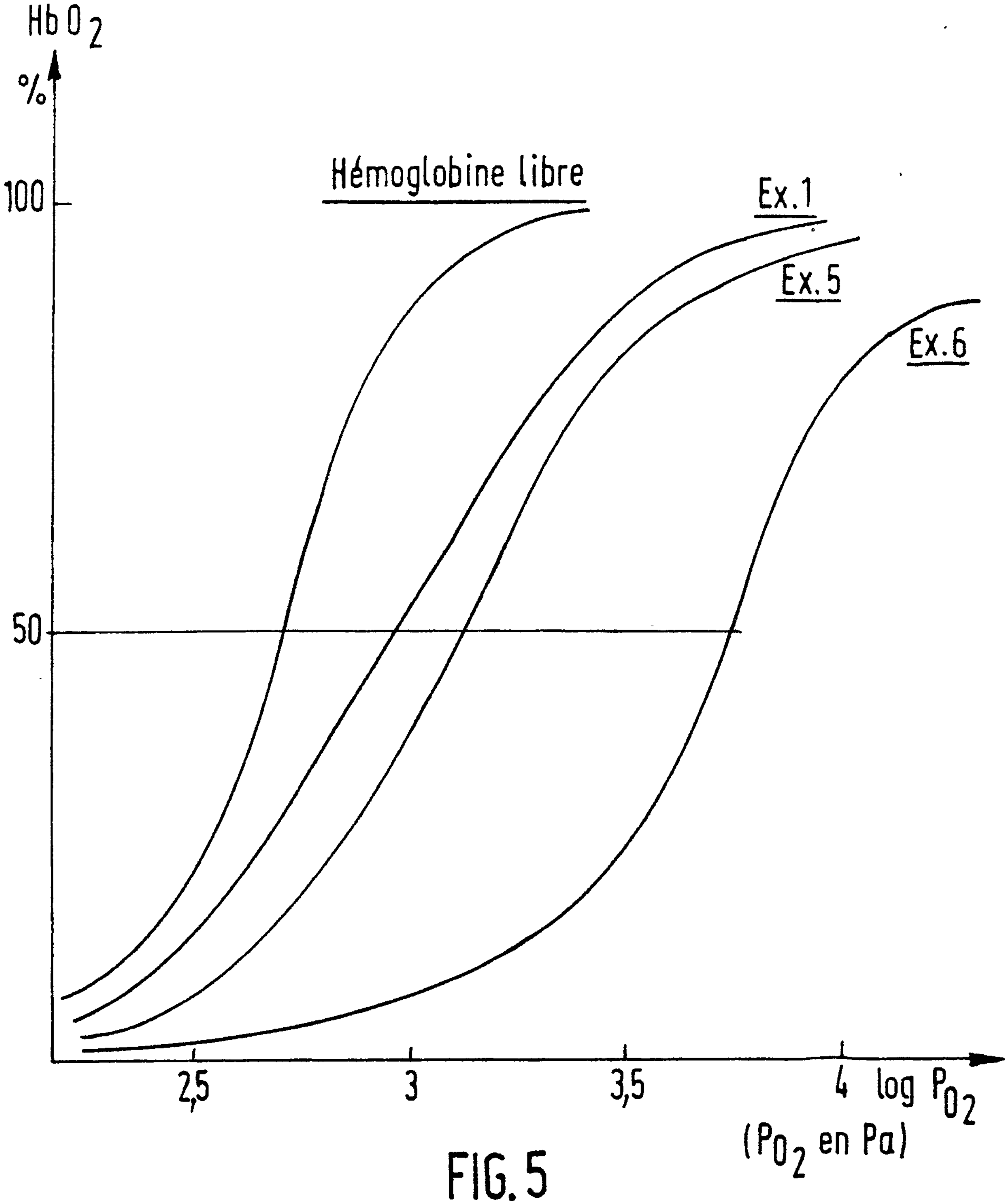

HbO2
%
100
50
Hémoglobine libre
Ex.1
Ex.5
Ex.6
2,5
3
3,5
4
log PO2
(PO2 en Pa)


FIG. 5

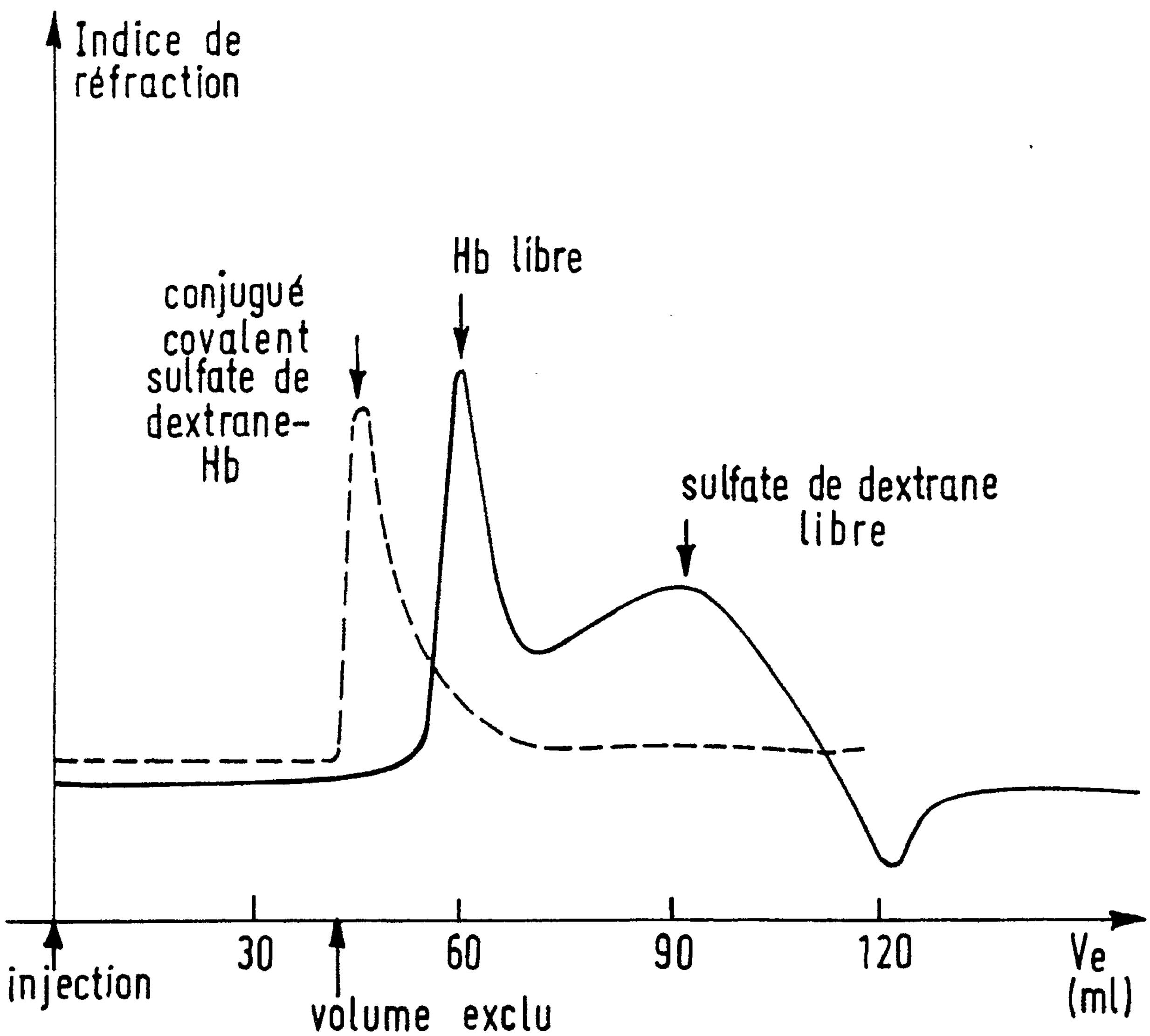
Indice de réfraction
conjugué covalent sulfate de dextrane-Hb
Hb libre
sulfate de dextrane libre
injection
30
volume exclu
60
90
120
Ve (ml)

FIG.6